(19) Europäisches Patentamt
European Patent Office
Office européen des brevets

(11) EP 1 981 559 B1

(12) **EUROPÄISCHE PATENTSCHRIFT**

(45) Veröffentlichungstag und Bekanntmachung des Hinweises auf die Patenterteilung:
**23.11.2016 Patentblatt 2016/47**

(21) Anmeldenummer: **07721910.3**

(22) Anmeldetag: **09.02.2007**

(51) Int Cl.:
***A61L 29/16*** (2006.01) ***A61L 29/18*** (2006.01)

(86) Internationale Anmeldenummer:
**PCT/DE2007/000242**

(87) Internationale Veröffentlichungsnummer:
**WO 2007/090385 (16.08.2007 Gazette 2007/33)**

# (54) FALTENBALLONBESCHICHTUNGSVERFAHREN

COATING METHOD FOR A FOLDED BALLOON

PROCÉDÉ DE REVÊTEMENT DE BALLONNET REPLIABLE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **09.02.2006 DE 102006006067**

(43) Veröffentlichungstag der Anmeldung:
**22.10.2008 Patentblatt 2008/43**

(73) Patentinhaber: **B. Braun Melsungen AG**
**34212 Melsungen (DE)**

(72) Erfinder:
- **VON HOLST, Armin**
**52080 Aachen (DE)**
- **HEITZMANN, Christoph**
**12459 Berlin (DE)**

(74) Vertreter: **Patentanwälte**
**Ruff, Wilhelm, Beier, Dauster & Partner mbB**
**Kronenstraße 30**
**70174 Stuttgart (DE)**

(56) Entgegenhaltungen:
**WO-A-2004/028582**
**US-A- 5 102 402**
**US-A- 5 370 614**
**US-A- 5 738 901**
**US-A1- 2003 130 717**

EP 1 981 559 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft Zusammensetzungen geeignet für die gezielte Befüllung oder gezielte Beschichtung von Falten eines Faltenballons und insbesondere Zusammensetzungen aus einem Kontrastmittel und einem Wirkstoff in einem Lösungsmittel zur Befüllung von Falten eines Katheterfaltenballons sowie Verfahren zur gezielten Befüllung oder gezielten Beschichtung der Falten eines Katheterfaltenballons und befüllte oder beschichtete Katheterfaltenballons erhältlich nach einem dieser Verfahren.

Zur Behandlung von Stenosen ist es Stand der Technik, Stents zu setzen oder durch sogenanntes biologisches Stenting die verengte Gefäßregion wieder aufzuweiten. Dazu werden oftmals Stents und/oder Katheterballons verwendet, welche mit einem Polymer als Wirkstoffträger und einem Anti-Restenose-Wirkstoff beschichtet sind.

Da in der Regel der Katheterballon mit oder ohne aufgesetztem, d.h. gekrimptem Stent eine längere Gefäßstrecke passieren muss, um zum Ort der Gefäßverengung zu gelangen, stellt sich das Problem, den Wirkstoff vor führzeitiger Ablösung von dem Stent und/oder Ballon zu schützen.

Bezüglich der Stents hat man bereits diverse Lösungsvorschläge für dieses Problem vorgeschlagen. So offenbaren US 2004/0071861 und WO 03/035131 A beispielsweise, dass in das Gittergerüst des Stent kleine Kavitäten zur Aufnahme eines Wirkstoffs vorgesehen werden können und man den Wirkstoff in den Kavitäten durch das Aufbringen einer Schutzbeschichtung schützen kann.

WO 02/45744 A beschreibt einen Stent ohne Kavitäten, der mit einer ersten Beschichtung umfassend einen Wirkstoff und einer darüber liegenden unelastischen Deckschicht versehen ist, welche den Wirkstoff schützt. Die Erfindung gemäß WO 02/45744 A bestehe nun darin, dass bei der Expansion des Stent die unelastische Deckschicht aufbricht und den Wirkstoff gezielt am Ort seiner Bestimmung freisetzt.

Für Katheterballons sind derartige Deckschichten auch denkbar, aufgrund der großen Fläche des Katheterballons jedoch als praktikable Ausführungsformen schwerer zu realisieren.

Das europäische Patent EP 0 519 063 B1 offenbart die Möglichkeit, einen Faltenballon mit Mikrokapseln zu beschichten, wobei in den Mikrokapseln ein pharmakologischer Wirkstoff eingeschlossen sein kann. Ferner offenbart EP 0 519 063 B1 die Möglichkeit, einen Teil der Mikrokapseln in den Falten des Katheterballon einzuschließen, wenn der Katheterballon im expandierten, d.h. im inflatierten Zustand mit den Mikrokapseln bestäubt und danach wieder zusammengefaltet, d.h. deflatiert wird (s. Anspruch 8 von EP 0 519 063 B1).

Diese Ausführungsform weist jedoch weiterhin das Problem auf, dass nur ein Teil der Mikrokapseln in den Falten eingeschlossen wird und somit die in den Falten befindliche Wirkstoffmenge nicht bekannt ist. Die nicht in den Falten eingeschlossenen Mikrokapseln werden beim Einführen des Katheterballons fast vollständig von der Ballonoberfläche gelöst und gelangen nicht zum Bestimmungsort. Zudem ist das Beschichtungsverfahren gemäß EP 0 519 063 B1 nur auf Feststoffe und insbesondere Mikrokapseln beschränkt und kann nicht auf Flüssigkeiten angewendet werden.

Aus der WO 2004/028582 A1 ist ein Beschichtungsverfahren für gefaltete Ballonkatheter bekannt, bei welchem die Katheter in Wirkstofflösungen eingetaucht werden. Dabei kann die Lösung auch kapillar unter die Falten eindringen. Es wird außerdem vorgeschlagen die außen haftende Lösung zu entfernen.

Die Aufgabe der vorliegenden Erfindung bestand darin, bei der Wirkstoffapplikation via Katheterballon den Wirkstoff derart aufzutragen, dass eine ungewünschte vorzeitige Ablösung nicht eintritt.

Diese Aufgabe wird durch die Bereitstellung eines Beschichtungsverfahrens gemäß Patentanspruch 1 gelöst. Weitere vorteilhafte Ausgestaltungen, Aspekte und Details der Erfindung ergeben sich aus den abhängigen Ansprüchen, der Beschreibung, den Beispielen und den Figuren.

Die Aufgabe wird durch die Bereitstellung eines Beschichtungsverfahrens gelöst, welches den Faltenballon im komprimierten oder nur minimal inflatierten Zustand beschichtet.

Dadurch ist die in die Falten eingebrachte Wirkstoffmenge genau bekannt, was für klinische Zulassungen ein wichtiger Aspekt ist und zudem können die Wirkstoffe bequem in gelöster Form eingebracht werden, ohne eine vorherige komplizierte Bereitstellung von speziellen pharmakologischen Formulierungen wie beispielsweise Mikrokapseln.

Das erfindungsgemäße Beschichtungsverfahren beschichtet gezielt die Falten, d.h. befüllt gezielt die Falten, wobei die restliche Ballonoberfläche, welche sich nicht unter den Falten befindet, unbeschichtet bleibt.

Die gezielte Befüllung der Falten erfolgt mittels eines sogenannten punktuellen Beschichtungsverfahrens. Nicht angewendet werden Tauchverfahren oder Sprühverfahren, welche die gesamte Ballonoberfläche abdecken. Zudem werden die erfindungsgemäßen Verfahren bei deflatierten und komprimierten oder nur geringfügig aufgeblasenen Katheterballons angewendet.

Grundsätzlich wird bei dem erfindungsgemäßen Verfahren jede Falte einzeln befüllt oder beschichtet. Bevor die nächste Falte befüllt oder beschichtet wird, kann der Inhalt der ersten Falte getrocknet werden, was jedoch nicht in jedem Fall erforderlich ist. Möglich ist auch, alle Falten nacheinander zu befüllen bzw. zu beschichten und danach den Inhalt aller Falten zu trocknen.

Erfindungsgemäß erfolgt die Trocknung unter Rotation des Katheterballon, worauf später noch genauer eingegangen wird.

Die Beschichtung oder Befüllung der Falten erfolgt mit einem flüssigen Gemisch umfassend zumindest ein Kontrastmittel, zumindest einen Wirkstoff und ein Lösungsmittel. Des weiteren können noch Salze, Hilfsstoffe oder andere pharmakologisch verträgliche Stoffe anwesend sein.

Als Wirkstoffe können beliebige antiproliferative, antiinflammatorische, antiphlogistische, zytostatische, zytotoxische, antiangiogene, antirestenotische oder antithrombotische Wirkstoffe eingesetzt werden.

Beispiele derartiger Wirkstoffe sind Abciximab, Acemetacin, Acetylvismion B, Aclarubicin, Ademetionin, Adriamycin, Aescin, Afromoson, Akagerin, Aldesleukin, Amidoron, Aminoglutethemid, Amsacrin, Anakinra, Anastrozol, Anemonin, Anopterin, Antimykotika, Antithrombotika, Apocymarin, Argatroban, Aristolactam-All, Aristolochsäure, Ascomycin, Asparaginase, Aspirin, Atorvastatin, Auranofin, Azathioprin, Azithromycin, Baccatin, Bafilomycin, Basiliximab, Bendamustin, Benzocain, Berberin, Betulin, Betulinsäure, Bilobol, Biolimus, Bisparthenolidin, Bleomycin, Bombrestatin, Boswellinsäuren und ihre Derivate, Bruceanole A, B und C, Bryophyllin A, Busulfan, Antithrombin, Bivalirudin, Cadherine, Camptothecin, Capecitabin, o-Carbamoylphenoxyessigsäure, Carboplatin, Carmustin, Celecoxib, Cepharantin, Cerivastatin, CETP-Inhibitoren, Chlorambucil, Chloroquinphosphat, Cictoxin, Ciprofloxacin, Cisplatin, Cladribin, Clarithromycin, Colchicin, Concanamycin, Coumadin, C-Type Natriuretic Peptide (CNP), Cudraisoflavon A, Curcumin, Cyclophosphamid, Cyclosporin A, Cytarabin, Dacarbazin, Daclizumab, Dactinomycin, Dapson, Daunorubicin, Diclofenac, 1,11-Dimethoxycanthin-6-on, Docetaxel, Doxorubicin, Dunaimycin, Epirubicin, Epothilone A und B, Erythromycin, Estramustin, Etobosid, Everolimus, Filgrastim, Fluroblastin, Fluvastatin, Fludarabin, Fludarabin-5'-dihydrogenphosphat, Fluorouracil, Folimycin, Fosfestrol, Gemcitabin, Ghalakinosid, Ginkgol, Ginkgolsäure, Glykosid 1 a, 4-Hydroxyoxycyclophosphamid, Idarubicin, Ifosfamid, Josamycin, Lapachol, Lomustin, Lovastatin, Melphalan, Midecamycin, Mitoxantron, Nimustin, Pitavastatin, Pravastatin, Procarbazin, Mitomycin, Methotrexat, Mercaptopurin, Thioguanin, Oxaliplatin, Irinotecan, Topotecan, Hydroxycarbamid, Miltefosin, Pentostatin, Pegasparase, Exemestan, Letrozol, Formestan, SMC-Proliferation-Inhibitor-2$\omega$, Mitoxanthrone, Mycophenolatmofetil, c-myc-Antisense, b-myc-Antisense, ß-Lapachon,Podophyllotoxin, Podophyllsäure-2-ethylhydrazid, Molgramostim (rhuGM-CSF), Peginterferon $\alpha$-2b, Lanograstim (r-HuG-CSF), Macrogol, Selectin (Cytokinantagonist), Cytokininhibitoren, COX-2-Inhibitor, NFkB, Angiopeptin, die Muskelzellproliferation hemmende monoklonale Antikörper, bFGF-Antagonisten, Probucol, Prostaglandine, 1-Hydroxy-11-Methoxycanthin-6-on, Scopolectin, NO-Donatoren, Pentaerythrityltetranitrat, Syndnoeimine, S-Nitrosoderivate, Tamoxifen, Staurosporin, ß-Estradiol, $\alpha$-Estradiol, Estriol, Estron, Ethinylestradiol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Paclitaxel, Derivate und Analog des Paclitaxel, 6-$\alpha$-Hydroxy-Paclitaxel, 2'-Succinylpaclitaxel, 2'-Succinylpaclitaxeltriethanolamin, 2'-Glutarylpaclitaxel, 2'-Glutarylpaclitaxeltriethanolamin, 2'-O-Ester von Paclitaxel mit N-(Dimethylaminoethyl)glutamid, 2'-O-Ester von Paclitaxel mit N-(Dimethylaminoethyl)glutamidhydrochlorid, Taxotere, Kohlensuboxids (MCS), macrocyclische Oligomere von Kohensuboxid, Mofebutazon, Lonazolac, Lidocain, Ketoprofen, Mefenaminsäure, Piroxicam, Meloxicam, Penicillamin, Hydroxychloroquin, Natriumaurothiomalat, Oxaceprol, ß-Sitosterin, Myrtecain, Polidocanol, Nonivamid, Levomenthol, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator Gewebsinhibitor der Metallproteinase-1 und 2, freie Nukleinsäuren, in Virenüberträger inkorporierte Nukleinsäuren, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika wie Cefadroxil, Cefazolin, Cefaclor, Cefotixin, Tobramycin, Gentamycin, Penicilline wie Dicloxacillin, Oxacillin, Sulfonamide, Metronidazol, Enoxoparin, desulfatiertes und N-reacetyliertes Heparin, Gewebe-Plasminogen-Aktivator, GpIlb/IIla-Plättchenmembranrezeptor, Faktor Xa-Inhibitor Antikörper, Heparin, Hirudin, r-Hirudin, PPACK, Protamin, Prourokinase, Streptokinase, Warfarin, Urokinase, Vasodilatoren wie Dipyramidol, Trapidil, Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Prostacyclin, Vapiprost, Interferon a, ß und Y, Histaminantagonisten, Serotoninblocker, Apoptoseinhibitoren, Apoptoseregulatoren wie p65, NF-kB oder Bcl-xL-Antisense-Oligonukleotiden, Halofuginon, Nifedipin, Tocopherol Tranilast, Molsidomin, Teepolyphenole, Epicatechingallat, Epigallocatechingallat, Leflunomid, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, natürliche und synthetisch hergestellte Steroide wie Inotodiol, Maquirosid A, Ghalakinosid, Mansonin, Streblosid, Hydrocortison, Betamethason, Dexamethason, nichtsteroidale Substanzen (NSAIDS) wie Fenoporfen, Ibuprofen, Indomethacin, Naproxen, Phenylbutazon und andere antivirale Agentien wie Acyclovir, Ganciclovir und Zidovudin, Clotrimazol, Flucytosin, Griseofulvin, Ketoconazol, Miconazol, Nystatin, Terbinafin, antiprozoale Agentien wie Chloroquin, Mefloquin, Quinin, des weiteren natürliche Terpenoide wie Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceantinoside C, Yadanzioside N, und P, Isodeoxyelephantopin, Tomenphantopin A und B, Coronarin A,B,C und D, Ursolsäure, Hyptatsäure A, Iso-Iridogermanal, Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, Cymarin, Hydroxyanopterin, Protoanemonin, Cheliburinchlorid, Sinococulin A und B, Dihydronitidin, Nitidinchlorid, 12-beta-Hydroxypregnadien 3,20-dion, Helenalin, Indicin, Indicin-N-oxid, Lasiocarpin, Inotodiol, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Periplocosid A, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Dihydrousambaraensin, Hydroxyusambarin, Strychnopentamin, Strychnophyllin, Usambarin, Usambarensin, Liriodenin, Oxoushinsunin, Daphnoretin, Lariciresinol, Methoxylariciresinol, Syringaresinol, Sirolimus (Rapamycin), Somatostatin, Tacrolimus, Roxithromycin, Troleandomycin, Simvastatin, Rosuvastatin, Vinblastin, Vincristin, Vindesin, Teniposid, Vinorelbin, Tropfosfamid, Treosulfan, Tremozolomid, Thiotepa, Tretinoin, Spiramycin, Umbelliferon, Desacetylvismion A, Vismion A und B, Zeorin sowie Gemische der vorgenannten Wirkstoffe.

Insbesondere bevorzugte Wirkstoffe sind Rapamycin (Sirolimus) und Paclitaxel, Derivate und Analog des Paclitaxel, 6-α-Hydroxy-Paclitaxel, 2'-Succinylpaclitaxel, 2'-Succinylpaclitaxeltriethanol-amin, 2'-Glutarylpaclitaxel, 2'-Glutarylpaclitaxeltriethanolamin, 2'-O-Ester von Paclitaxel mit N-(Dimethylaminoethyl)glutamid, 2'-O-Ester von Paclitaxel mit N-(Dimethylaminoethyl)glutamidhydrochlorid, Taxotere sowie Gemische der vorgenannten Wirkstoffe. Insbesondere bevorzugt ist Paclitaxel.

Natürlich können auch Wirkstoffgemische eingesetzt werden wie beispielsweise ein Wirkstoffgemisch aus Paclitaxel und Trapidil oder aus Paclitaxel mit einem NO-Donor oder aus Rapamycin mit Vitamin A oder Vitamin C.

Auch wichtig ist das verwendete Lösungsmittel, welches nach Befüllung der Falten durch Trocknung bei Normaldruck oder im Vakuum entfernt wird. Mindestens ein Alkohol ist als Lösungsmittel vorhanden.

Als Lösungsmittel können leichtflüchtige organische Verbindungen verwendet werden wie beispielsweise Ethanol, Methanol, Propanol. Bei der Wahl des Lösungsmittel ist es vor allem wichtig, dass es das Material des Katheterballons nicht angreift bzw. unbrauchbar macht bzw. die Kontaktzeit des Lösungsmittels so gering ist, dass keine Schädigung entstehen kann bzw. eine eventuelle Schädigung unerheblich ist und nicht zu Schädigungen führt, welche bei der Dilatation ein Platzen des Ballons verursachen können.

Als Lösungsmittel haben sich Alkohole und insbesondere Diole und Triole auch in Kombination mit Monoolen bewährt. Das bevorzugte Lösungsmittel kann vorzugsweise aus folgender Gruppe ausgewählt werden: Methanol, Ethanol, Iospropanol, n-Butanol, iso-Butanol, t-Butanol, Ethylenglykol, Propylenglykol, 1,3-Propandiol, Butylenglykol, 1,3-Butandiol, 1,4-Butandiol, Glycerin, 1,2,3-Butantriol, 1,2,4-Butantriol und 1,2,3,4-Butantetraol, wobei auch Gemische dieser Lösungsmittel sowie Gemische mit den vorher genannten organischen Lösungsmitteln eingesetzt werden können. Bevorzugt sind Ethylenglykol, Propylenglykol, 1,3-Propandiol, Butylenglykol, 1,3-Butandiol, 1,4-Butandiol und insbesondere bevorzugt ist Glycerin.

Auch Wasser kann im Lösungsmittelgemisch enthalten sein, jedoch vorzugsweise in einer Menge geringer als 50 Gew.-%, vorzugsweise 30 Gew.-% und insbesondere bevorzugt 10 Gew.-% bezogen auf die Gesamtlösung.

Je nach erfindungsgemäßem Beschichtungsverfahren werden dünnviskose Lösungen des Wirkstoffs oder dickviskose Lösungen des Wirkstoffs oder der Wirkstoffkombination benötigt.

Viskositätsmessungen sind für einen Fachmann gängige Praxis und erfolgen vorzugsweise mit Viskosimetern. Die Viskosität muss je nach Art und Ausgestaltung der Falten eingestellt werden.

Diverse Faltentypen und Typen und Ausführungsformen von Faltenballons sind beispielsweise in EP 0 519 063 B1, WO 94/23787 A1 oder WO 03/059430 A1 offenbart. Da jedoch fast jeder deflatierbare bzw. dilatierbare Katheterballon Falten aufweist, ist das erfindungsgemäße Beschichtungsverfahren grundsätzlich auf jeden aufblasbaren Katheterballon und nicht nur auf die in WO 94/23787 A1 oder WO 03/059430 A1 genannten speziellen Ausführungsformen beschränkt.

Als Lösungsmittel können neben den oben genannten oder zusammen mit den oben genannten Lösungsmitteln auch Öle, Fettsäuren und Fettsäureester verwendet werden. Bevorzugte Öle sind beispielsweise: Leinöl, Flachsöl, Hanföl, Maiskeimöl, Walnussöl, Rapsöl, Sojaöl, Sonnenblumenöl, Mohnöl, Safloröl (Färberdistelöl), Weizenkeimöl, Distelöl, Traubenkernöl, Nachtkerzenöl, Borretschöl, Schwarzkümmelöl, Algenöl, Fischöl, Lebertran und/oder Mischungen der vorgenannten Öle eingesetzt.

Unverdünnte Öle werden dann als Lösungsmittel eingesetzt, wenn eine dickviskose Beschichtungslösung benötigt wird. Durch Zugabe lipophiler Lösungsmittel kann die Viskosität erniedrigt und somit ein gewünschter Viskositätsgrad eingestellt werden. Andererseits können dünnviskose Lösungen durch Zugabe von Ölen oder Fetten in ihrer Viskosität erhöht werden.

Der Begriff "Lösung" oder "Beschichtungslösung" wie hierin verwendet soll nicht nur klare Lösungen bezeichnen sondern auch Emulsionen, Dispersionen und Suspensionen von einem oder mehreren Wirkstoffen in einem Lösungsmittel oder Lösungsmittelgemisch optional mit weiteren Trägern, Hilfsstoffen oder beispielsweise Kontrastmitteln.

Der Begriff "Zusammensetzung" oder "wirkstoffenthaltende Zusammensetzung" wie hierin verwendet soll nicht nur Lösungen bezeichnen sondern auch Emulsionen, Dispersionen, Suspensionen, Öle, Pasten und dickflüssige Mischungen enthaltend mindestens eine pharmakologischen Wirkstoff. Dennoch sind diese Zusammensetzungen nicht fest, sondern dünnflüssig bis dickflüssig oder gel- oder pastenförmig.

Als Öle oder grundsätzlich als lipophile Substanzen können natürliche und synthetische Öle, Fette, Lipide, Lipoide und Wachse eingesetzt werden.

WO 03/022265 A1 beschreibt beispielsweise ölige Formulierungen von Paclitaxel, welche ebenfalls eingesetzt werden können und bevorzugt sind. Vergleichbare Öllösungen lassen sich auch mit anderen Wirkstoffen wie beispielsweise Trapidil oder Rapamycin herstellen.

Weitere Beispiele für geeignete Öle oder lipophile Substanzen lassen sich durch folgende allgemeinen Formeln darstellen:

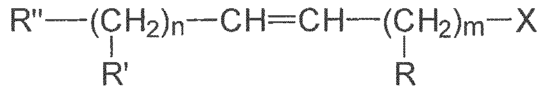

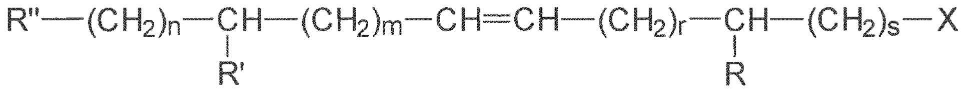

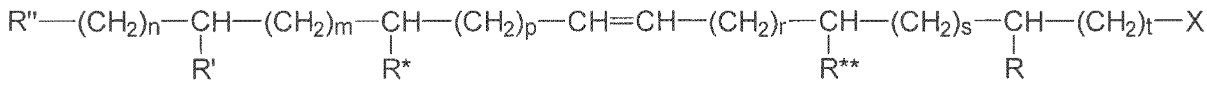

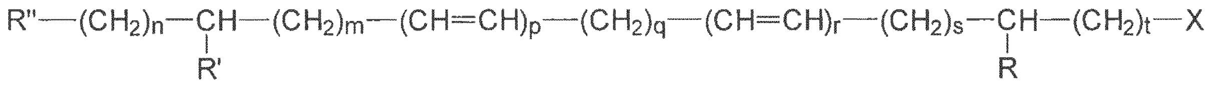

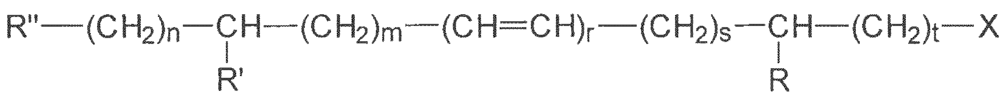

worin

R, R', R", R* und R** unabhängig voneinander für Alkyl-, Alenyl-, Alkinyl-, Heteroalkyl-, Cycloalkyl-, Heterocyclylreste mit 1 bis 20 Kohlenstoffatomen, Aryl-, Arylalkyl-, Alyklaryl-, Heteroarylreste mit 3 bis 20 Kohlenstoffatomen oder für funktionelle Gruppen stehen und bevorzugt folgende Reste bedeuten: -H, -OH, $-OCH_3$, $-OC_2H_5$, $-OC_3H_7$, -O-cyclo-$C_3H_5$, $-OCH(CH_3)_2$, $-OC(CH_3)_3$, $-OC_4H_9$, -OPh, $-OCH_2$-Ph, $-OCPh_3$, -SH, $-SCH_3$, $-SC_2H_5$, $-NO_2$, -F, -Cl, -Br, -I, -CN, -OCN, -NCO, -SCN, -NCS, -CHO, $-COCH_3$, $-COC_2H_5$, $-COC_3H_7$, -CO-cyclo-$C_3H_5$, $-COCH(CH_3)_2$, $-COC(CH_3)_3$, -COOH, $-COOCH_3$, $-COOC_2H_5$, $-COOC_3H_7$, -COO-cyclo-$C_3H_5$, $-COOCH(CH_3)_2$, $-COOC(CH_3)_3$, -OOC-$CH_3$, -OOC-$C_2H_5$, -OOC-$C_3H_7$, -OOC-cyclo-$C_3H_5$, -OOC-$CH(CH_3)_2$, -OOC-$C(CH_3)_3$, $-CONH_2$, $-CONHCH_3$, $-CONHC_2H_5$, $-CONHC_3H_7$, $-CON(CH_3)_2$, $-CON(C_2H_5)_2$, $-CON(C_3H_7)_2$, $-NH_2$, $-NHCH_3$, $-NHC_2H_5$, $-NHC_3H_7$, -NH-cyclo-$C_3H_5$, $-NHCH(CH_3)_2$, $-NHC(CH_3)_3$, $-N(CH_3)_2$, $-N(C_2H_5)_2$, $-N(C_3H_7)2$, -N(cyclo-$C_3H_5)_2$, $-N[CH(CH_3)_2]_2$, $-N[C(CH_3)_3]_2$, $-SOCH_3$, $-SOC_2H_5$, $-SOC_3H_7$, $-SO_2CH_3$, $-SO_2C_2H_5$, $-SO_2C_3H_7$, $-SO_3H$, $-SO_3CH_3$, $-SO_3C_2H_5$, $-SO_3C_3H_7$, $-OCF_3$, $-OC_2F_5$, -O-$COOCH_3$, -O-$COOC_2H_5$, -O-$COOC_3H_7$, -O-COO-cyclo-$C_3H_5$, -O-$COOCH(CH_3)_2$, -O-$COOC(CH_3)_3$, -NH-CO-$NH_2$, -NH-CO-$NHCH_3$, -NH-CO-$NHC_2H_5$, -NH-CO-$N(CH_3)_2$, -NH-CO-$N(C_2H_5)_2$, -O-CO-$NH_2$, -O-CO-$NHCH_3$, -O-CO-$NHC_2H_5$, -O-CO-$NHC_3H_7$, -O-CO-$N(CH_3)_2$, -O-CO-$N(C_2H_5)_2$, -O-CO-$OCH_3$, -O-CO-$OC_2H_5$, -O-CO-$OC_3H_7$, -O-CO-O-cyclo-$C_3H_5$, -O-CO-$OCH(CH_3)_2$, -O-CO-$OC(CH_3)_3$, $-CH_2F$, $-CHF_2$, $-CF_3$, $-CH_2Cl$, $-CH_2Br$, $-CH_2I$, $-CH_2$-$CH_2F$, $-CH_2$-$CHF_2$, $-CH_2$-$CF_3$, $-CH_2$-$CH_2Cl$, $-CH_2$-$CH_2Br$, $-CH_2$-$CH_2I$, $-CH_3$, $-C_2H_5$, $-C_3H_7$, -cyclo-$C_3H_5$, $-CH(CH_3)_2$, $-C(CH_3)_3$, $-C_4H_9$, $-CH_2$-$CH(CH_3)_2$, $-CH(CH_3)$-$C_2H_5$, -Ph, $-CH_2$-Ph, $-CPh_3$, $-CH=CH_2$, $-CH_2$-$CH=CH_2$, $-C(CH_3)=CH_2$, -CH=CH-$CH_3$, $-C_2H_4$-$CH=CH_2$, $-CH=C(CH_3)_2$, -C=CH, -C=C-$CH_3$, $-CH_2$-C=CH;

X für eine Estergruppe oder Amidgruppe und insbesondere für -O-alkyl, -O-CO-alykl, -O-CO-O-alkyl, -O-CO-NH-alkyl, -O-CO-N-dialkyl, -CO-NH-alkyl, -CO-N-dialkyl, -CO-O-alkyl, -CO-OH, -OH;

m, n, p, q, r, s und t unabhängig voneinander ganze Zahlen von 0 bis 20, bevorzugt von 0 bis 10 bedeuten.

Die Bezeichnung "alkyl" beispielsweise bei -CO-O-alkyl bedeutet vorzugsweise eine der für die vorgenannten Reste R, R' usw. genannten Alkylreste, z.B. $-CH_2$-Ph. Die Verbindungen der vorgenannten allgemeinen Formeln können auch in Form ihrer Salze, als Racemate oder Diastereomerengemische, als reine Enantiomeren oder Diastereomeren sowie als Gemische oder Oligomere oder Copolymere oder Blockcopolymere vorliegen. Ferner können die vorgenannten Verbindungen im Gemisch mit anderen Substanzen wie den biostabilen und biodegradierbaren Polymeren und insbesondere im Gemisch mit den hierin genannten Ölen und/oder Fettsäuren eingesetzt werden. Bevorzugt sind derartige Gemische und Einzelsubstanzen, welche zur Polymerisation, insbesondere zur Autopolymerisation geeignet sind.

Bevorzugt sind jedoch natürlich vorkommende Öle, Fettsäuren und Fettsäureester wie beispielsweise Ölsäure, Eicosapentaensäure, Timnodonsäure, Docosahexaensäure, Arachidonsäure, Linolsäure, α-Linolensäure, Y-Linolensäure sowie Mischungen und Ester der vorgenannten Fettsäuren. Insbesondere bevorzugt sind Omega-9-Fettsäuren, Omega-3-Fettsäuren und Omega-6-Fettsäuren sowie deren Ester und Mischungen enthaltend diese Stoffe vorzugsweise mit einem Gewichtsanteil von mindestens 10 Gew.-%.
Weitere geeignete Fettsäuren sind in den Tabellen 1 bis 4 aufgeführt.

Tabelle 1: Monoolefinische Fettsäuren

| Systematischer Name | Trivialname | Kurzform |
|---|---|---|
| cis-9-Tetradecensäure | Myristoleinsäure | 14:1(n-5) |
| cis-9-Hexadecensäure | Palmitoleinsäure | 16:1(n-7) |
| cis-6-Octadecensäure | Petroselinsäure | 18:1(n-12) |
| cis-9-Octadecensäure | Ölsäure | 18:1(n-9) |
| cis-11-Octadecensäure | Vaccensäure | 18:1(n-7) |
| cis-9-Eicosensäure | Gadoleinsäure | 20:1(n-11) |
| cis-11-Eicosensäure | Gondoinsäure | 20:1(n-9) |
| cis-13-Docosensäure | Erucinsäure | 22:1(n-9) |
| cis-15-Tetracosensäure | Nervonsäure | 24:1(n-9) |
| t9-Octadecensäure | Elaidinsäure | |
| t11-Octadecensäure | t-Vaccensäure | |
| t3-Hexadecensäure | | trans-16:1 n-13 |

Tabelle 2: Polyungesättigte Fettsäuren

| Systematischer Name | Trivialname | Kurzform |
|---|---|---|
| 9,12-Octadecadiensäure | Linolsäure | 18:2(n-6) |
| 6,9,12-Octadecatriensäure | γ-Linolsäure | 18:3(n-6) |
| 8,11,14-Eicosatriensäure | Dihomo-γ-linolensäure | 20:3(n-6) |
| 5,8,11,14-Eicosatetraensäure | Arachidonsäure | 20:4(n-6) |
| 7,10,13,16-Docosatetraensäure | | 22:4(n-6) |
| 4,7,10,13,16-Docosapentaensäure | - | 22:5(n-6) |
| 9,12,15-Octadecatriensäure | α-Linolensäure | 18:3(n-3) |
| 6,9,12,15-Octadecatetraensäure | Stearidonsäure | 18:4(n-3) |
| 8,11,14,17-Eicosatetraensäure | - | 20:4(n-3) |
| 5,8,11,14,17-Eicosapentaensäure | EPA | 20:5(n-3) |
| 7,10,13,16,19-Docosapentaensäure | DPA | 22:5(n-3) |
| 4,7,10,13,16,19-Docosahexaensäure | DHA | 22:6(n-3) |
| 5,8,11-Eicosatriensäure | Meadsäure | 20:3(n-9) |
| 9c 11t 13t Eleostearinsäure | | |
| 8t 10t 12c Calendinsäure | | |
| 9c 11t 13c Catalpicsäure | | |
| 4, 7, 9, 11, 13, 16, 19 Docosaheptadecansäure | Stellaheptaensäure | |
| | Taxolsäure | all-cis-5,9-18:2 |

(fortgesetzt)

| Systematischer Name | Trivialname | Kurzform |
|---|---|---|
| | Pinolensäure | all-cis-5,9,12-18:3 |
| | Sciadonsäure | all-cis-5,11,14-20:3 |

Tabelle 3: Acetylenische Fettsäuren

| Systematischer Name | Trivialname |
|---|---|
| 6-Octadecinsäure | Taririnsäure |
| t11-Octadecen-9-insäure | Santalbin- oder Ximeninsäure |
| 9-Octadecinsäure | Stearolinsäure |
| 6-Octadecen-9-insäure | 6,9-Octadeceninsäure~ |
| t10-Heptadecen-8-insäure | Pyrulinsäure |
| 9-Octadecen-12-insäure | Crepenynsäure |
| t7,t11-Octadecadiene-9-insäure | Heisterinsäure |
| t8,t10-0ctadecadiene-12-insäure | |
| 5,8,11,14-Eicosatetrainsäure | ETYA |

Tabelle 4: gesättigte Fettsäuren

| Systematischer Name | Trivialname | Kurzform |
|---|---|---|
| Dodecansäure | Laurinsäure | 12:0 |
| Tetradecansäure | Myristinsäure | 14:0 |
| Hexadecansäure | Palmitinsäure | 16:0 |
| Heptadecansäure | Margarinsäure | 17:0 |
| Octadecansäure | Stearinsäure | 18:0 |
| Eicosansäure | Arachinsäure | 20:0 |
| Docosansäure | Behensäure | 22:0 |
| Tetracosansäure | Lignocerinsäure | 24:0 |

Des weiteren sind die Ester der in den Tabellen 1 - 4 aufgeführten Fettsäuren und insbesondere deren Ethylester und Gemische enthaltend diese Fettsäuren und/oder Fettsäureester bevorzugt. Weitere bevorzugte Fettsäuren sind 6,8-Dithianoctansäure, $\gamma$-Linolensäure und $\alpha$-Liponsäure sowie deren Ester.

Des weiteren sind als Zusatzstoffe in der einzubringenden Zusammensetzung auch Kontrastmittel enthalten.

Als Matrix zur Aufnahme des Wirkstoffs in den Falten des Katheterballons haben sich niedermolekulare Verbindungen und insbesondere Kontrastmittel, Kontrastmittelanaloga oder Kontrastmittel-ähnliche Verbindungen als geeignet herausgestellt. Als Kontrastmittelanaloga oder Kontrastmittel-ähnliche Verbindungen werden Stoffe bezeichnet, welche nicht mit der Bezeichnung "Kontrastmittel" betitelt werden, jedoch die Eigenschaften eines Kontrastmittels besitzen, nämlich durch bildgebende Verfahren und Diagnoseverfahren dargestellt zu werden. Bei diesen Verbindungen handelt es sich zumeist um Stoffe, welche Barium, lod, Mangan, Eisen, Lanthan, Cer, Praseodym, Neodym, Samarium, Europium, Gadolinium, Terbium, Dysprosium, Holmium, Erbium, Thulium, Ytterbium und/oder Lutetium enthalten.

Als Kontrastmittel können übliche Kontrastmittel für Röntgenaufnahmen, Computertomographie (CT), Kernspintomographie oder Magnetresonanztomographie (MRT) eingesetzt werden.

Prinzipiell zu unterscheiden sind Kontrastmittel, die bei Röntgenuntersuchungen zum Einsatz kommen (Röntgenkontrastmittel), und die, die bei magnetresonanztomographischen Untersuchungen eingesetzt werden (MR-Kontrastmittel), wobei die Röntgenkontrastmittel wie z.B. Jod-Lipiodol® bevorzugt sind.

Im Falle von Röntgenkontrastmitteln handelt es sich um Substanzen, die entweder zu einer vermehrten Absorption einfallender Röntgenstrahlen gegenüber der umgebenden Struktur führen (sog. positive Kontrastmittel) oder einfallende Röntgenstrahlen vermehrt ungehindert durchlassen (sog. negative Kontrastmittel).

Des weiteren sind iodhaltige Kontrastmittel bevorzugt, welche bei der Gefäßdarstellung (Angiographie und Phlebographie) und bei der CT (Computertomographie) verwendet werden.

Insbesondere bevorzugt sind Kontrastmittel mit einem 1,3,5-Trüodbenzolkern, nephrotrope niederosmolare Röntgenkontrastmittel, Amidotrizoesäure, Iothalaminsäure, Iotrolan, Iopamidol, Iodoxaminsäure, Diatrizoesäure, Iomeprol, Iopromid, Desmethoxyacetyl-Iopromid (DAMI) oder 5-Amino-2,4,6-triiodphthalsäure-(2,3-dihydroxypropyl)-amid (ATH).

Im folgenden sind einige Strukturen der insbesondere bevorzugten Kontrastmittel gezeigt:

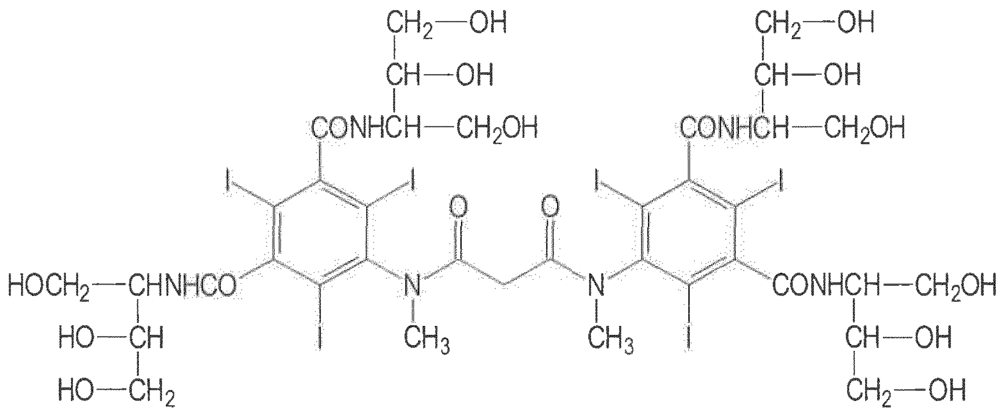

Iotrolan

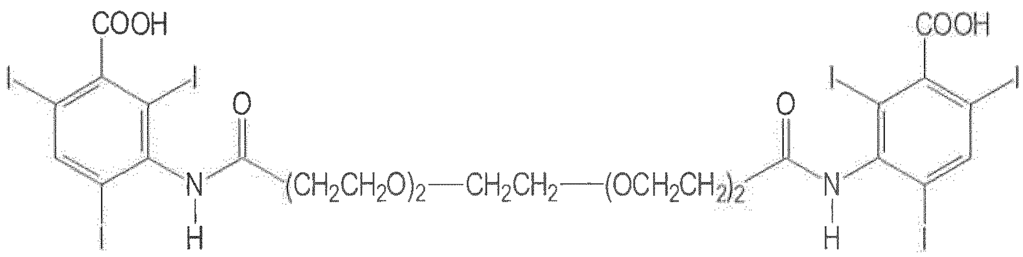

Iodoxaminsäure

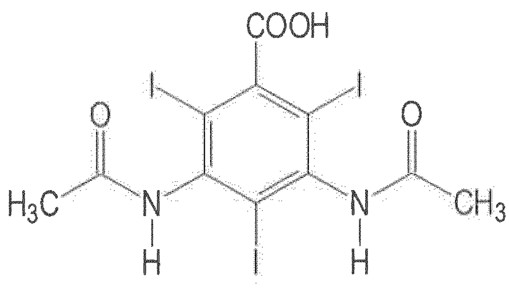

Amidotrizoesäure

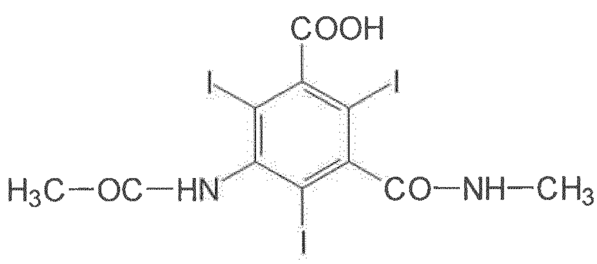

Iothalaminsäure

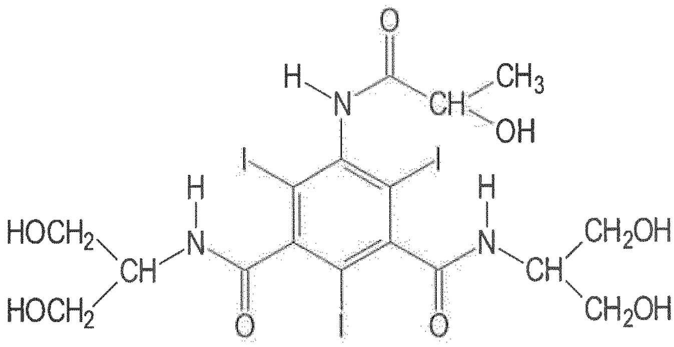

Iopamidol

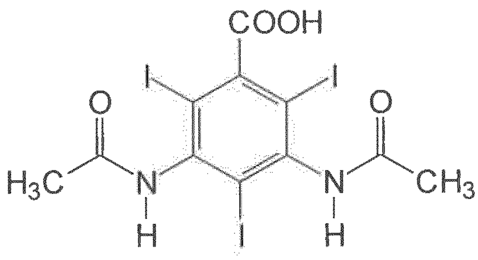

Diatrizoesäure

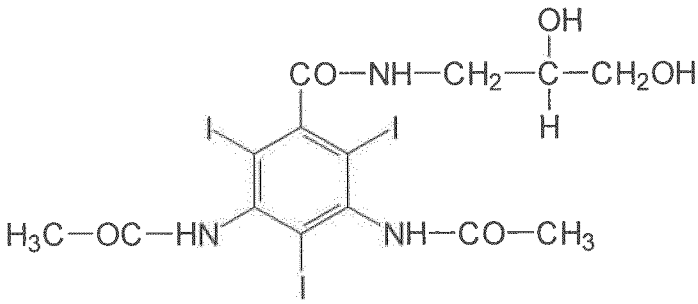

Iomeprol

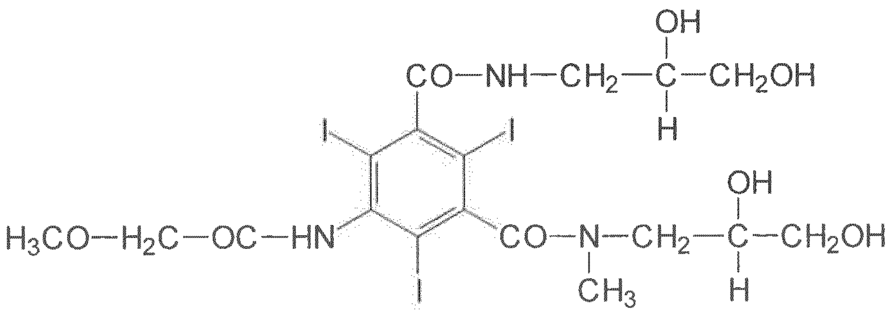

Iopromid

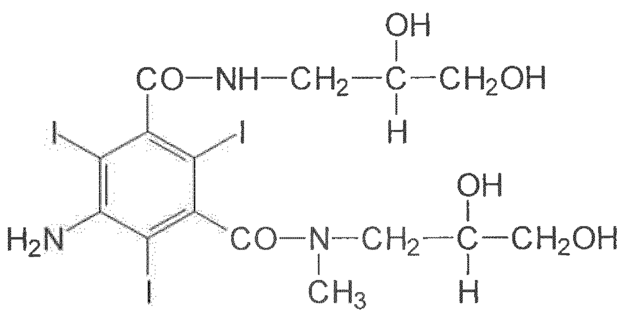

Desmethoxyacetyl-Iopromid (DAMI)

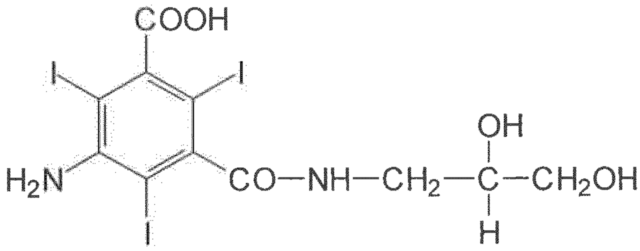

5-Amino-2,4,6-triiodphthalsäure-(2,3-dihydroxypropyl)-amid (ATH)

Eine weitere Klasse von bevorzugten Kontrastmitteln stellen die paramagnetischen Kontrastmittel dar, welche zumeist ein Lanthanoid enthalten.

Zu den paramagnetischen Substanzen, die über ungepaarte Elektronen verfügen, zählt z.B. das Gadolinium ($Gd^{3+}$), das insgesamt sieben ungepaarte Elektronen besitzt. Des weiteren gehören zu dieser Gruppe das Europium ($Eu^{2+}$, $Eu^{3+}$), Dysprosium ($Dy^{3+}$) und Holmium ($Ho^{3+}$). Diese Lanthanoide können auch in chelatisierter Form unter Verwendung von beispielsweise Hämoglobin, Chlorophyll, Polyazasäuren, Polycarbonsäuren und insbesondere EDTA, DTPA sowie DOTA als Chelatbildner eingesetzt werden. Beispiele für Gadolinium-haltige Kontrastmittel sind Gadolinium-Diethylentriaminpentaessigsäure, Gadopentetsäure (GaDPTA), Gadodiamid, Meglumin-Gadoterat oder Gadoteridol

Erfindungsgemäß können natürlich auch Mischungen zweier oder mehrerer Kontrastmittel eingesetzt werden.

Des weiteren können auch physiologisch verträgliche Salze in der Zusammensetzung des Wirkstoffs oder des Wirkstoffgemisches anwesend sein. Bevorzugt sind Lösungen oder Dispersionen eines Wirkstoffs vorzugsweise Paclitaxel oder Rapamycin und insbesondere Paclitaxel zusammen mit einem oder mehreren physiologisch verträglichen Salzen.

Als Salze können bevorzugt Verbindungen enthaltend Natriumkationen, Calcium-, Magnesium-, Zink-, Eisen- oder Lithiumkationen zusammen mit Sulfat-, Chlorid-, Bromit-, Iodid-, Phosphat-, Nitrat-, Citrat- oder Acetatanionen eingesetzt werden. Als Salze können zudem auch ionische Kontrastmittel eingesetzt werden oder ionische Kontrastmittel können den oben genannten Salzen zugesetzt werden.

Anstelle oder in Kombination mit den vorgenannten Salzen können auch Aminosäuren, Oligopeptide, Polyaminosäuren, Peptide und/oder Vitamine eingesetzt werden.

Geeignete Aminosäuren sind: Glycin, Alanin, Valin, Leucin, Isoleucin, Serin, Threonin, Phenylalanin, Tyrosin, Tryptophan, Lysin, Arginin, Histidin, Aspartat, Glutamat, Asparagin, Glutamin, Cystein, Methionin, Prolin, 4-Hydroxyprolin, N,N,N-Trimethyllysin, 3-Methylhistidin, 5-Hydroxylysin, O-Phosphoserin, $\gamma$-Carboxyglutamat, $\varepsilon$-N-Acetyttysin, $\omega$-N-Methytarginin, Citrullin, Ornithin und Derivate dieser Aminosäuren.

Geeignete Vitamine umfassen: Vitamin A, Vitamin C (Ascorbinsäure), Vitamin D, Vitamin H, Vitamin K, Vitamin E, VitaminB1, VitaminB2, VitaminB3, VitaminB5, VitaminB6, VitaminB12, Thiamin, Riboflavin, Niacin, Pyridoxin und Folsäure.

Ferner sind auch liposomale Formulierungen des Wirkstoffs oder Wirkstoffgemisches für die Beschichtung bzw. Befüllung von Katheterballons einsetztbar.

Die liposomalen Formulierungen werden vorzugsweise hergestellt, indem in einem ersten Schritt der Wirkstoff (z.B. Paclitaxel oder Rapamycin) bzw. die Wirkstoffkombination in einem wässrigen Medium oder Puffermedium gelöst und anschließend mit Lösungen, die membranbildende Substanzen enthalten, in Kontakt gebracht wird. Bei diesem Verfahren ergeben sich hohe Einschlussraten von mindestens 30% bis zu 95%.

Membranbildende Substanzen sind geladene amphiphile Verbindungen bevorzugt Alkylcarbonsäuren, Alkylsulfonsäuren, Alkylamine, Alkylammoniumsalze, Phosphorsäureester mit Alkoholen, natürliche sowie synthetische Lipide, wie Phosphatidylglycerol (PG), Phosphatidylserin (PS), Derivate des Phophatidylethanolamins (PE-Derivate) sowie des Cholesterols, Phosphatidsäure, Phosphatidylinositol, Cardiolipin, Sphingomyelin, Ceramid in seinen natürlichen, halb-synthetischen oder synthetischen Formen, Stearylamin und Stearinsäure, Palmitoyl-D-glucuronid und/oder geladene Sphingolipide, wie z. B. Sulfatid.

Als neutrale membranbildende Substanzen fungieren an sich bekannte Komponenten wie z. B. Phosphatidylcholin (PC), Phophatidylethanolamin (PE), Steroide, vorzugsweise Cholesterol, komplexe Lipide und/oder neutrale Sphingolipide.

Die Gewinnung der Liposomen aus der wässrigen Lösung erfolgt ebenfalls nach an sich bekannten Techniken so z. B. durch Dialyse, Ultrafiltration, Gelfiltration, Sedimentation oder Flotation. Die Liposomen besitzen einen durchschnittlichen Durchmesser von 10 bis 400 nm.

Derartige liposomale Formulierungen lassen sich auch bevorzugt in die Falten eines Faltenballons mittels Kapillarverfahren (Pipettierverfahren) einbringen.

Das erfindungsgemäße Faltenbeschichtungsverfahren oder Faltenbefüllungsverfahren ist das Pipettierverfahren auch als Kapillarverfahren bezeichnet.

Somit betrifft die vorliegende Erfindung ein Verfahren zur Beschichtung oder Befüllung der Falten eines Katheterfaltenballons auf folgende Weise:

a) eine wirkstoffenthaltende Zusammensetzung am distalen oder proximalen Ende einer Falte des Katheterfaltenballons abgegeben und die Falte aufgrund von Kapillarkräften befüllt wird.

Vorteilhaft ist, dass dieses Beschichtungs- oder Befüllungsverfahren vorzugsweise im komprimierten oder deflatierten Zustand oder maximal 10% inflatierten Zustand des Katheterballons durchgeführt wird. Unter dem Begriff "10% inflatierten Zustand" soll verstanden werden, dass der Katheterballon 10% der Inflation, d.h. der Aufdehnung bis zur bei der Dilatation geplanten Maximalausdehnung erfahren hat. Wird die bei der Dilatation vorgesehene Aufdehnung mit 100% bezeichnet und der deflatierte Zustand mit 0% angesetzt, so ergibt sich eine 10%ige Inflation gemäß folgender Formel:

$$\text{(Durchmesser des Katheterballons im deflatierten Zustand)} + \text{(Durchmesser des Katheterballons im inflatierten Zustand – Durchmesser des Katheterballons im deflatierten Zustand)} / 10$$

Ferner können gemäß dem erfindungsgemäßen Verfahren mehrere oder alle Falten gleichzeitig beschichtet oder befüllt werden und die Beschichtung oder Befüllung kann gezielt erfolgen. Eine gezielte Befüllung der Falten oder gezielte Beschichtung der Falten soll heißen, dass nur die Falten befüllt oder beschichtet werden und die Oberfläche des Katheterfaltenballons außerhalb der Falten nicht beschichtet wird.

Im folgenden wird auf das erfindungsgemäße Beschichtungs- und Befüllungsverfahren genauer eingegangen.

Pipettierverfahren oder Kapillarverfahren:

Bei diesem Verfahren wird eine Pipette oder Spritze oder eine andere zur punktförmigen Abgabe der wirkstoffenthaltenden Zusammensetzung befähigte Vorrichtung eingesetzt.

Unter dem Begriff "wirkstoffenthaltende Zusammensetzung" wie hierin verwendet wird das Gemisch aus Wirkstoff und Lösungsmittel und/oder Hilfsstoff und/oder Träger verstanden, also eine tatsächliche Lösung, Dispersion, Suspension oder Emulsion aus einem Wirkstoff oder einem Wirkstoffgemisch und mindestens einem weiteren Bestandteil ausgewählt aus den hierin genannten Lösungsmitteln, Ölen, Fettsäuren, Fettsäureester, Aminosäuren, Vitaminen, Kontrastmitteln, Salzen und/oder membranbildenden Substanzen. Der Begriff "Lösung" soll ferner verdeutlichen, dass es sich um ein flüssiges Gemisch handelt, welches jedoch auch zähflüssig (dickviskos oder hochviskos) sein kann.

Die Pipette oder Spritze oder die andere zur punktförmigen Abgabe der wirkstoffenthaltenden Zusammensetzung befähigte Vorrichtung wird mit der Zusammensetzung befüllt und am proximalen oder am distalen Ende einer Falte angesetzt. Die austretende Zusammensetzung wird aufgrund von Kapillarkräften in die Falte und entlang der Falte gezogen bis das gegenüberliegende Ende der Falte erreicht ist.

Der Katheterballon befindet sich im komprimierten, d.h. deflatierten Zustand. Eine auch nur teilweise oder geringfügige Inflation des Katheterballons ist in der Regel nicht erforderlich um die Falten ein wenig zu öffnen. Dennoch kann die Befüllung der Falten bei einer geringfügigen Inflation des Katheterballons von maximal 10% des bei der Dilatation vorgesehenen Durchmessers erfolgen. Die Befüllung der Falten kann zudem bei einer leichten Aufweitung der Falten erfolgen indem 100 kPa (1 bar) Überdruck, vorzugsweise 50 kPa (0,5 bar) Überdruck angelegt werden, um die Falten leicht aufzuweiten.

Bei diesem Verfahren ist es wichtig, dass die wirkstoffenthaltende Zusammensetzung dünnflüssig genug ist, um die entsprechenden Kapillarkräfte zu entwickeln.

Als Zusammensetzungen werden insbesondere Lösungen von einem Wirkstoff oder Wirkstoffgemisch in einem Alkohol oder Alkoholgemisch bevorzugt.

Die Kapillarkräfte sollten derart stark sein, dass sich eine Falte einer Länge von 10 mm innerhalb von 5 bis 80 Sekunden, vorzugsweise innerhalb von 15 bis 60 Sekunden und insbesondere bevorzugt innerhalb von 25 bis 45 Sekunden vollständig füllt.

Ist die Zusammensetzung bzw. Lösung zu viskos kann es zudem von Vorteil sein, den Katheterballon mit der zu befüllenden Falte nach oben aus der horizontalen Lage um maximal 45°, vorzugsweise maximal 30° zu neigen und dadurch auch die Schwerkraft mit zu nutzen. In der Regel erfolgt die Befüllung einer Falte mittels Kapillarkräften jedoch im horizontalen Zustand des Katheterballons mit der zu befüllenden Falte nach oben. Die Spritze oder Pipette oder die

andere zur punktförmigen Abgabe der wirkstoffenthaltenden Zusammensetzung befähigte Vorrichtung wird vorzugsweise am proximalen oder am distalen Ende der Falte spitz in Richtung des Faltenverlaufs in einem Winkel von 10° bis 65°, vorzugsweise 20° bis 55°, weiter bevorzugt in einem Winkel von 27° bis 50° und insbesondere bevorzugt in einem Winkel von 35° bis 45° gemessen von der Horizontalen an die Falte angesetzt.

Hat die Zusammensetzung zum Befüllen der Falten bzw. der vorliegenden Falte das gegenüberliegende Ende erreicht, so stoppt der Substanzfluß in der Regel von selbst und die Spritze oder Pipette oder die andere zur punktförmigen Abgabe der wirkstoffenthaltenden Zusammensetzung befähigte Vorrichtung kann entfernt werden.

Damit nun kein größerer Tropfen an wirkstoffenthaltender Zusammensetzung am Ansatzpunkt der Spritze oder Pipette oder der anderen zur punktförmigen Abgabe der wirkstoffenthaltenden Zusammensetzung befähigten Vorrichtung zurückbleibt, hat sich als vorteilhaft erwiesen, die Spritze oder Pipette oder die andere Abgabevorrichtung bereits zu entfernen, bevor die wirkstoffenthaltende Zusammensetzung vollständig bis an das andere Ende der Falte gelangt ist. Dadurch wird auch noch die restliche am Ansatzpunkt der Spritze oder der Pipette oder der anderen Abgabevorrichtung verbliebene wirkstoffenthaltende Zusammensetzung in die Falte hineingezogen, so dass keine Beschichtungszusammensetzung oder besser Befüllungszusammensetzung außerhalb der Falte verbleibt.

Vorzugsweise wird die Spritze oder Pipette oder die andere Abgabevorrichtung dann entfernt, wenn ca. 90% der Falte mit wirkstoffenthaltender Zusammensetzung befüllt sind. Der optimale Zeitpunkt zur Entfernung der Spritze oder Pipette oder der anderen Abgabevorrichtung kann durch wenige Versuche genau bestimmt werden und ist auch reproduzierbar.

Unter dem Begriff "eine andere zur punktförmigen Abgabe der wirkstoffenthaltenden Zusammensetzung befähigte Vorrichtung" bezeichnet eine Vorrichtung, welche ähnlich einer Pipette befähig ist, einen gleichmäßigen und kontinuierlichen Fluß an wirkstoffenthaltender Zusammensetzung zu liefern, so dass darunter auch eine Pumpe, Mikropumpe oder ein anderes Reservoir verstanden werden kann, welches diese gleichmäßige und kontinuierliche Abgabe an wirkstoffenthaltender Zusammensetzung gewährleistet.

Nach dem Befüllen einer Falte wird der Katheterballon gedreht, so dass die nächste zu beschichtende Falte nach oben und vorzugsweise horizontal liegt. Der Faltenbefüllungsvorgang wird nun wiederholt.

Je nach Konsistenz der wirkstoffenthaltenden Zusammensetzung kann es notwendig sein, die vorher befüllte Falte zu trocknen bevor der Ballon gedreht wird, um die nächste Falte zu befüllen. Die Trocknung erfolgt vorzugsweise durch Verdunstung des Lösungsmittels.

Ferner ist es bei diesem Verfahren auch möglich zwei, mehr als zwei oder alle Falten eines Katheterballons gleichzeitig zu befüllen oder zu beschichten, falls die Konsistenz der wirkstoffenthaltenden Zusammensetzung dies zulässt, d.h. die Konsistenz nicht derart dünnflüssig ist, dass die Zusammensetzung aus den nicht horizontal liegenden Falten ausläuft.

Insbesondere das Pipettierverfahren eignet sich zur gleichzeitigen Befüllung mehrerer oder aller Falten eines Katheterballons. Dabei kann der Katheterballon waagerecht oder vorzugsweise senkrecht gelagert werden und die Abgabevorrichtungen werden von oben an die Enden der Falten vorzugsweise im Winkel von 10 bis 70 Grad angesetzt, so dass die wirkstoffenthaltende Zusammensetzung in die Falten fließen kann.

Wurden alle Falten des Ballons befüllt, so erfolgt die Endtrocknung. Grundsätzlich ist es natürlich nicht erforderlich, dass alle Falten des Katheterfaltenballons befüllt werden, wobei jedoch die Befüllung aller Falten die gängige und bevorzugte Ausführungsform ist, da bei der Dilatation in möglichst kurzer Zeit eine größtmögliche Menge an Wirkstoff auf die Gefäßwand übertragen werden soll.

Bei den erfindungsgemäßen Faltenballons dauert die Dilatation vorzugsweise maximal 60 Sekunden und insbesondere bevorzugt maximal 30 Sekunden.

Nach der Befüllung der letzten Falte erfolgt die Trocknung der letzten Falten, d.h. des Inhalts der letzten Falte vorzugsweise ohne Vakuum unter Normaldruck durch Verdunstung des Lösungsmittels.

An diese Vortrocknung kann sich eine Endtrocknung anschließen, welche erfindungsgemäß bei rotierendem Katheterballon durchgeführt wird. Falls erforderlich oder gewünscht kann zudem noch Vakuum während der Rotation angelegt werden. Dieses besondere Trocknungsverfahren wird im Anschluß an die erfindungsgemäßen Beschichtungsverfahren eingehender beschrieben.

Rotationstrocknuna:

Wie oben erwähnt können die beschichteten oder befüllten Katheterballons nach dem Befüllen oder Beschichten jeder Falte oder nach der Beschichtung oder Befüllung aller Falten oder der zu beschichtenden bzw. zu befüllenden Falten, falls nicht alle Falten beschichtet oder befüllt werden sollen, im rotierenden Zustand getrocknet werden.

Diese Rotationstrocknung hat mehrere Vorteile. Zum einen wird dadurch die wirkstoffenthaltende Zusammensetzung getrocknet und zudem gleichmäßig in den Falten als auch auf der Oberfläche innerhalb der Falten verteilt.

Die Rotationstrocknung ist insbesondere bei öligen oder zähflüssigen wirkstoffenthaltenden Zusammensetzung geeignet, eine gleichmäßige Verteilung der Zusammensetzung in der jeweiligen Falte zu erreichen.

Zudem kann bei der Rotation des Katheterballons Vakuum angelegt werden, um eine intensive Trocknung der wirkstoffenthaltenden Zusammensetzung zu erreichen.

Bei der Trocknung im Vakuum treten gerade bei zähflüssigen, hochviskosen oder in den festen Zustand übergehenden Lösungen Siedeverzüge auf, d.h. in dem Öl oder Feststoff eingeschlossene Lösungsmittelreste werden spontan freigesetzt und zerreißen oder sprengen die Beschichtung oder Befüllung. Durch eine Trocknung im Vakuum bei gleichzeitiger Rotation wird werden diese Siedeverzüge vermieden und es wird eine getrocknete gleichmäßige Beschichtung der inneren Oberfläche der Falten erfalten.

Zudem ist die Drehrichtung der Rotation entscheidend. Die Drehrichtung erfolgt in Richtung der Faltenöffnungen, wenn man dies aus dem Inneren der Falte betrachtet. In Figur 2 ist die Drehrichtung angezeigt und der Katheterballon wird so wie ein Schaufelrad einer Schaufelradbaggers gedreht, damit die wirkstoffenthaltende Zusammensetzung aufgrund der Rotationskraft in das Falteninnere gedrückt wird.

Vorzugsweise wird der Faltenballon mit einer Rotationsgeschwindigkeit von 50 bis 500, vorzugsweise 150 bis 300 Umdrehungen pro Minute gedreht.

Das Pipettierverfahren eignet sich bevorzugt für leichtviskose, mittelviskose bis leicht zähviskose Zusammensetzungen.

Der Begriff Viskosizität bezieht sich auf die dynamische Viskosität [η]:

$$[\eta] = \frac{\text{kg}}{\text{m} \cdot \text{s}} = \text{Pa} \cdot \text{s} = \frac{\text{Ns}}{\text{m}^2}$$

Das Pipettierverfahren kann vorzugsweise bei mittelviskosen Zusammensetzungen eingesetzt werden. Bevorzugt sind Viskositäten bei Raumtemperatur im Bereich von vorzugsweise 0,5 mPa s bis 5000 mPa s, weiter bevorzugt im Bereicht von 0,7 mPa s bis 1000 mPa s, noch weiter bevorzugt im Bereicht von 0,9 mPa s bis 200 mPa s und insbesondere bevorzugt im Bereicht von 1,0 mPa s bis 100 mPa s. In diesem Viskositätsbereich liegen Zusammensetzungen aus Ölen, Kontrastmitteln und/oder Salzen, welche mit üblichen Lösungsmitteln insbesondere Alkoholen verdünnt sind. Das Pipettierverfahren kann über einen sehr breiten Viskositätsbereich angewendet werden.

Überraschenderweise wurde ferner gefunden, dass eine Zusammensetzung umfassend mindestens ein Kontrastmittel und mindestens einen antiproliferativen, antiinflammatorischen, antiphlogistischen, zytostatischen, zytotoxischen, antiangiogenen, anti-restenotischen oder antithrombotischen Wirkstoff und mindestens einen Alkohol als Lösungsmittel sich für die Beschichtung bzw. Befüllung der Falten von Katheterfaltenballons besonders gut eignet.

Es wurde bewußt kein Polymer als Wirkstoffträger eingesetzt, welches durch das erfindungsgemäße Beschichtungsverfahren nur schwer in die Falten des Katheterballons einzubringen war, die Falten teilweise verklebte, so dass bei der Dilatation des Katheterfaltenballons eine ungleichmäßige Öffnung der Falten und eine nicht einheitliche Wirkstoffabgabe eintrat.

Hat die einzubringende Mischung oder Lösung eine Konsistenz, so dass sie in die Falten hineinfließen kann, dann wird der Faltenballon mit einer Falte noch oben horizontal gelagert oder vorzugsweise um 5 bis 25 Grad geneigt, so dass die Spritze oder Düse am unteren Ende des Faltenballons an der Faltenöffnung angesetzt werden kann und die Mischung eigenständig in die Falte hineinfließt und diese voll ausfüllt.

Bei diesen Salzlösungen mit hohem Salzgehalt wird vorzugsweise Wasser als Lösungsmittel eingesetzt, weil Wasser das Ballonmaterial nicht angreift und beschädigt. Sobald die Mischung eine Konsistenz hat, dass sie nicht mehr aus der Falte herausfließen kann, wird der Faltenballon gedreht und die nächste Falte wird befüllt bis in der Regel alle 4 bis 6 Falten des Ballons befüllt sind. Faltenballons werden bevorzugt im komprimierten Zustand beschichtet, wobei einige spezielle Ausführungsformen von Faltenballons auch im expandierten Zustand beschichtet werden können.

Ein solches Beschichtungsverfahren umfasst die Schritte

a) Bereitstellen eines Faltenballons
b) Platzierung einer Falte des Ballons in einer horizontalen oder bis zu 25 Grad geneigten Position,
c) Ansetzen der Spritzenöffnung an die dem Kopfende des Ballons zugewandten Faltenöffnung,
d) Ausführung einer Relativbewegung von Spritzenöffnung und Faltenballon in Längsrichtung der Falte,
e) Befüllung der Falte während des Bewegungsvorgangs mit einer Mischung aus einem Wirkstoff und einem Salz und/oder einem ionischen Kontrastmittel in einem geeigneten Lösungsmittel,
f) sofern erforderlich Trocknung der in der Falte befindlichen Mischung bis zu einem Grad, der ein Auslaufen der Mischung aus der Falte verhindert,
g) Drehung des Ballons um 360° dividiert durch die Anzahl der Falten,
h) Wiederholung der Schritte b) bis g) bis alle Falten befüllt sind und
i) Trocknung der Mischungen in des Falten bis sich die Mischung verfestigt.

Werden dünnflüssigere Mischungen eingesetzt, so wird bei Schritt c) die Spritzenöffnung an das Fußende angesetzt und ohne Relativbewegung gemäß Schritt d) die Falte vorwiegend aufgrund von Kapillarkräften befüllt.

## Figurenbeschreibung

Fig. 1 zeigt einen vollständig in allen vier Falten befüllten Katheterfaltenballon im nicht expandierten Zustand, wobei die in den Falten befindliche Zusammensetzung während der Rotation des Faltenballons getrocknet bzw. das Lösungsmittel entfernt wird.

## Beispiele

### Beispiel 1:

Paclitaxel (250 mg) oder Rapamycin (250 mg) oder eine Kombination aus Paclitaxel (150 mg) und Rapamycin (150 mg) werden in einem wässrigen Medium mit einem Ethanolanteil von ca. 5 Vol.-% und bei einem pH-Wert von 4 bis 5 gelöst oder suspendiert und anschließend mit einer Lösung aus Phosphatidylserin und Phosphatidylcholin im Gewichtsverhältnis 50 : 50 versetzt.

Es bildet sich eine liposomale Formulierung bei der der Wirkstoff oder die Wirkstoffkombination in den Vesikeln eingeschlossen ist.

Die Gewinnung der Liposomen aus der wässrigen Lösung erfolgt mittels Ultrafiltration, wobei eine Gewinnung der Liposomen nicht unbedingt zwingend ist.

Die gewonnenen Liposome oder die liposomale wässrige Formulierung wird in eine Kavität gefüllt und mittels des oben beschriebenen Pipettierverfahrens in eine Falte gefüllt, wobei eine Kapillare im Winkel von ca. 40 Grad an das distale Ende der Falte angesetzt. Der Katheterballon liegt dabei horizontal und die zu beschichtende Falt liegt oben. Aufgrund von Kapillarkräften zieht sich die Zusammensetzung innerhalb von ca. 48 Sekunden in die Falte. Bevor die Zusammensetzung das proximale Ende der Falte erreicht hat, wird die Kapillare entfernt, so dass auch noch die am Ansatzpunkt der Kapillare vorhandene Zusammensetzung in die Falte gezogen wird.

Der beschichtete Katheterballon wird danach an der Luft getrocknet und anschließend mittels des oben beschriebenen Rotationstrocknungsverfahren abschließend getrocknet.

### Beispiel 2:

Eine Lösung aus 250 μg Paclitaxel in einem Gemisch aus Ethanol, n-Butanol und Ethylenglykol im Volumenverhältnis 50 : 25 : 25 wird angesetzt und zu dieser Lösung werden 200 μg Vitamin A gegeben.

Diese Zusammensetzung wird dann in eine Abgabevorrichtung mit insgesamt 4 Kanülen gefüllt. Jede dieser vier Kanülen wird im spitzen Winkel von ca. 30 Grad an eine der vier Falten eines Katheterfaltenballons angesetzt und die vier Falten werden gleichzeitig mittels des oben beschriebenen Pipettierverfahrens befüllt.

Nach der Befüllung wird der Katheterballon langsam in Rotation versetzt und zeitgleich wird Vakuum angelegt und die Rotationsgeschwindigkeit mit steigendem Vakuum kurzzeitig bis auf 1.200 Umdrehungen gesteigert und der Falteninhalt wird gemäß dem oben beschriebenen Rotationstrocknungsverfahren getrocknet.

### Beispiel 3:

500μg Paclitaxel wird in 1 ml wasserfreiem Ethanol und 0,4 ml Essigsäure gelöst und mittels des oben beschriebenen Pipettierverfahrens in die Falten eines Katheterballon gefüllt.

Die anschließende Trocknung erfolgt nach Verdunstung des Lösungsmittels gemäß dem oben beschriebenen Rotationstrocknungsverfahren.

Nach der Beschichtung und Trocknung befinden sich in jeder Falte ca. 10 μg Paclitaxel.

### Beispiel 4:

200 μg Paclitaxel werden in 0,5 ml Ethanol und 0,5 ml DMSO und 0,1 ml Essigsäure gelöst. Ferner wird eine Lösung von 350 μg Kaliumacetal in Ethanol - Wasser im Volumenverhältnis 90 : 10 bereitgestellt. Die Kaliumacetatlösung wird nun zur Paclitaxellösung gegeben und die Lösungsmittel können verdunsten, bis die ersten Bestandteile beginnen auszufallen oder die Zusammensetzung beginnt sich zu trüben oder eine mittelviskose Zusammensetzung entsteht.

Diese Zusammensetzung wird mittels dem oben beschriebenen Pipettierverfahren in die Falten des Katheterballons gegeben. Erfindungsgemäß können die Falten einzeln und nacheinander befüllt oder auch gleichzeitig zusammen befüllt werden. Nach Verdunstung des Lösungsmittels erfolgt die finale Trocknung gemäß dem Rotationstrocknungsverfahren wie eingangs beschrieben.

**Beispiel 5:**

Die Lösung aus Beispiel 10a wird durch Zugabe von Propylenglykol und/oder Glycerin auf eine Viskosität von $10^3$ bis $10^4$ mPa·s eingestellt.

Die so erhaltene Zusammensetzung wird gemäß dem oben beschriebenen Pipettierverfahren in die Falten eingebracht werden, wobei die Falten einzeln nacheinander oder gleichzeitig zusammen befüllt werden können.

Die abschließende Trocknung erfolgt vorzugsweise mittels des oben beschriebenen Rotationstrocknungsverfahrens.

## Patentansprüche

**1.** Verfahren zur Beschichtung oder Befüllung der Falten eines Katheterfaltenballons umfassend die folgenden Schritte:

a) Bereitstellung einer Zusammensetzung umfassend mindestens ein Kontrastmittel und mindestens einen antiproliferativen, antiinflammatorischen, antiphlogistischen, zytostatischen, zytotoxischen, antiangiogenen, antirestenotischen oder antithrombotischen Wirkstoff und mindestens einen Alkohol als Lösungsmittel;
b) Einbringen der Zusammensetzung umfassend mindestens ein Kontrastmittel und mindestens einen antiproliferativen, antiinflammatorischen, antiphlogistischen, zytostatischen, zytotoxischen, antiangiogenen, antirestenotischen oder antithrombotischen Wirkstoff und mindestens einen Alkohol als Lösungsmittel mittels einer Spritzvorrichtung gezielt in die Falten des Katheterfaltenballons, wobei die Zusammensetzung aus der Spritzvorrichtung am distalen oder proximalen Ende einer Falte des Katheterballons abgegeben und die Falte aufgrund von Kapillarkräften befüllt wird;
c) Trocknung der Zusammensetzung umfassend mindestens ein Kontrastmittel und mindestens einen antiproliferativen, antiinflammatorischen, antiphlogistischen, zytostatischen, zytotoxischen, antiangiogenen, antirestenotischen oder antithrombotischen Wirkstoff und mindestens einen Alkohol als Lösungsmittel durch Entfernung des Lösungsmittels durch Verdunstung oder unter vermindertem Druck.

**2.** Verfahren gemäß Anspruch 1, wobei Katheterfaltenballons im komprimierten oder deflatierten Zustand oder maximal 10% inflatierten Zustand beschichtet oder befüllt wird.

**3.** Verfahren gemäß Anspruch 1 oder 2, wobei zwei oder mehr oder alle Falten des Katheterfaltenballons gleichzeitig befüllt oder beschichtet werden.

**4.** Verfahren gemäß einem der Ansprüche 1 - 3, wobei gezielt nur die Falten des Katheterfaltenballons beschichtet oder befüllt werden und die Oberfläche des Katheterfaltenballons außerhalb der Falten nicht beschichtet wird.

**5.** Verfahren gemäß einem der Ansprüche 1 - 4, wobei nach der Befüllung oder Beschichtung einer oder mehrerer Falten der Katheterfaltenballon in Richtung der Faltenöffnungen rotiert wird.

**6.** Verfahren gemäß einem der Ansprüche 1 - 5, wobei die Zusammensetzung mindestens einen Alkohol als Lösungsmittel enthält, ausgewählt aus der Gruppe umfassend:

Methanol, Ethanol, Iospropanol, n-Butanol, iso-Butanol, t-Butanol, Ethylenglykol, Propylenglykol, 1,3-Propandiol, Butylenglykol, 1,3-Butandiol, 1,4-Butandiol, Glycerin, 1,2,3-Butantriol, 1,2,4-Butantriol und 1,2,3,4-Butantetraol.

**7.** Verfahren gemäß einem der Ansprüche 1 - 6, wobei die Zusammensetzung mindestens ein Kontrastmittel enthält, ausgewählt aus der Gruppe umfassend:

Röntgenkontrastmittel, iodhaltige Kontrastmittel, Kontrastmittel mit einem 1,3,5-Triiodbenzolkern, nephrotrope niederosmolare Röntgenkontrastmittel, Amidotrizoesäure, Iothalaminsäure, Iotrolan, Iopamidol, Iodoxaminsäure, Diatrizoesäure, Iomeprol, Iopromid, Desmethoxyacetyl-Iopromid (DAMI), 5-Amino-2,4,6-triiodphthalsäure-(2,3-dihydroxypropyl)-amid (ATH).

**8.** Verfahren gemäß einem der Ansprüche 1 - 7, wobei die Zusammensetzung mindestens ein Öl und/oder mindestens eine Fettsäure enthält, ausgewählt aus der Gruppe umfassend:

Leinöl, Flachsöl, Hanföl, Maiskeimöl, Walnussöl, Rapsöl, Sojaöl, Sonnenblumenöl, Mohnöl, Safloröl (Färberdistelöl), Weizenkeimöl, Distelöl, Traubenkernöl, Nachtkerzenöl, Borretschöl, Schwarzkümmelöl, Algenöl, Fischöl, Lebertran, Taririnsäure, Ximeninsäure, Stearolinsäure, 6,9-Octadeceninsäure, Pyrulinsäure, Crepenynsäure, Heisterinsäure, ETYA, Linolsäure, γ-Linolsäure, Dihomo-γ-linolensäure, Arachidonsäure, α-Linolensäure, Stearidonsäure, EPA, DPA, DHA, Meadsäure, Stellaheptaensäure, Taxolsäure, Pinolensäure, Sciadonsäure, Myristoleinsäure, Palmitoleinsäure, Petroselinsäure, Ölsäure, Vaccensäure, Gadoleinsäure, Gondoinsäure, Erucinsäure, Nervonsäure, Elaidinsäure, t-Vaccensäure, Laurinsäure, Myristinsäure, Palmitinsäure, Margarinsäure, Stearinsäure, Arachinsäure, Behensäure, Lignocerinsäure, 6,8-Dithianoctansäure, γ-Linolensäure, α-Liponsäure.

**9.** Verfahren gemäß einem der Ansprüche 1 - 5, wobei der mindestens eine antiproliferative, antiinflammatorische, antiphlogistische, zytostatische, zytotoxische, antiangiogene, antirestenotische oder antithrombotische Wirkstoff aus der Gruppe ausgewählt wird umfassend:

Abciximab, Acemetacin, Acetylvismion B, Aclarubicin, Ademetionin, Adriamycin, Aescin, Afromoson, Akagerin, Aldesleukin, Amidoron, Aminoglutethemid, Amsacrin, Anakinra, Anastrozol, Anemonin, Anopterin, Antimykotika, Antithrombotika, Apocymarin, Argatroban, Aristolactam-All, Aristolochsäure, Ascomycin, Asparaginase, Aspirin, Atorvastatin, Auranofin, Azathioprin, Azithromycin, Baccatin, Bafilomycin, Basiliximab, Bendamustin, Benzocain, Berberin, Betulin, Betulinsäure, Bilobol, Biolimus, Bisparthenolidin, Bleomycin, Bombrestatin, Boswellinsäuren und ihre Derivate, Bruceanole A, B und C, Bryophyllin A, Busulfan, Antithrombin, Bivalirudin, Cadherine, Camptothecin, Capecitabin, o-Carbamoylphenoxyessigsäure, Carboplatin, Carmustin, Celecoxib, Cepharantin, Cerivastatin, CETP-Inhibitoren, Chlorambucil, Chloroquinphosphat, Cictoxin, Ciprofloxacin, Cisplatin, Cladribin, Clarithromycin, Colchicin, Concanamycin, Coumadin, C-Type Natriuretic Peptide (CNP), Cudraisoflavon A, Curcumin, Cyclophosphamid, Cyclosporin A, Cytarabin, Dacarbazin, Daclizumab, Dactinomycin, Dapson, Daunorubicin, Diclofenac, 1,11-Dimethoxycanthin-6-on, Docetaxel, Doxorubicin, Dunaimycin, Epirubicin, Epothilone A und B, Erythromycin, Estramustin, Etobosid, Everolimus, Filgrastim, Fluroblastin, Fluvastatin, Fludarabin, Fludarabin-5'-dihydrogenphosphat, Fluorouracil, Folimycin, Fosfestrol, Gemcitabin, Ghalakinosid, Ginkgol, Ginkgolsäure, Glykosid 1 a, 4-Hydroxyoxycyclophosphamid, Idarubicin, Ifosfamid, Josamycin, Lapachol, Lomustin, Lovastatin, Melphalan, Midecamycin, Mitoxantron, Nimustin, Pitavastatin, Pravastatin, Procarbazin, Mitomycin, Methotrexat, Mercaptopurin, Thioguanin, Oxaliplatin, Irinotecan, Topotecan, Hydroxycarbamid, Miltefosin, Pentostatin, Pegasparase, Exemestan, Letrozol, Formestan, SMC-Proliferation-Inhibitor-2ω, Mitoxanthrone, Mycophenolatmofetil, c-myc-Antisense, b-myc-Antisense, ß-Lapachon,Podophyllotoxin, Podophyllsäure-2-ethylhydrazid, Molgramostim (rhuGM-CSF), Peginterferon α-2b, Lanograstim (r-HuG-CSF), Macrogol, Selectin (Cytokinantagonist), Cytokininhibitoren, COX-2-Inhibitor, NFkB, Angiopeptin, die Muskelzellproliferation hemmende monoklonale Antikörper, bFGF-Antagonisten, Probucol, Prostaglandine, 1-Hydroxy-11-Methoxycanthin-6-on, Scopolectin, NO-Donatoren, Pentaerythrityltetranitrat, Syndnoeimine, S-Nitrosoderivate, Tamoxifen, Staurosporin, ß-Estradiol, α-Estradiol, Estriol, Estron, Ethinylestradiol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Paclitaxel, Derivate und Analog des Paclitaxel, 6-α-Hydroxy-Paclitaxel, 2'-Succinylpaclitaxel, 2'-Succinylpaclitaxeltriethanolamin, 2'-Glutarylpaclitaxel, 2'-Glutarylpaclitaxeltriethanolamin, 2'-O-Ester von Paclitaxel mit N-(Dimethylaminoethyl)glutamid, 2'-O-Ester von Paclitaxel mit N-(Dimethylaminoethyl)glutamidhydrochlorid, Taxotere, Kohlensuboxids (MCS), macrocyclische Oligomere von Kohensuboxid, Mofebutazon, Lonazolac, Lidocain, Ketoprofen, Mefenaminsäure, Piroxicam, Meloxicam, Penicillamin, Hydroxychloroquin, Natriumaurothiomalat, Oxaceprol, β-Sitosterin, Myrtecain, Polidocanol, Nonivamid, Levomenthol, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator Gewebsinhibitor der Metallproteinase-1 und 2, freie Nukleinsäuren, in Virenüberträger inkorporierte Nukleinsäuren, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika, Cefadroxil, Cefazolin, Cefaclor, Cefotixin, Tobramycin, Gentamycin, Penicilline, Dicloxacillin, Oxacillin, Sulfonamide, Metronidazol, Enoxoparin, desulfatiertes und N-reacetyliertes Heparin, Gewebe-Plasminogen-Aktivator, GpIIb/IIIa-Plättchenmembranrezeptor, Faktor Xa-Inhibitor Antikörper, Heparin, Hirudin, r-Hirudin, PPACK, Protamin, Prourokinase, Streptokinase, Warfarin, Urokinase, Vasodilatoren, Dipyramidol, Trapidil, Nitroprusside, PDGF-Antagonisten, Triazolopyrimidin, Seramin, ACE-Inhibitoren, Captopril, Cilazapril, Lisinopril, Enalapril, Losartan, Thioproteaseinhibitoren, Prostacyclin, Vapiprost, Interferon a, ß und Y, Histaminantagonisten, Serotoninblocker, Apoptoseinhibitoren, Apoptoseregulatoren, p65, NF-kB, Bcl-xL-Antisense-Oligonukleotiden, Halofuginon, Nifedipin, Tocopherol Tranilast, Molsidomin, Teepolyphenole, Epicatechingallat, Epigallocatechingallat, Leflunomid, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, natürliche und synthetisch hergestellte Steroide, Inotodiol, Maquirosid A, Ghalakinosid, Mansonin, Streblosid, Hydrocortison, Betamethason, Dexamethason, nichtsteroidale Substanzen (NSAIDS), Fenoporfen, Ibuprofen, Indomethacin, Naproxen, Phenylbutazon, antivirale Agentien, Acyclovir, Ganciclovir, Zidovudin, Clotrimazol, Flucytosin, Griseofulvin, Ketoconazol, Miconazol, Nystatin, Terbinafin, antiprozoale Agentien, Chloroquin, Mefloquin, Quinin, natürliche Terpenoide, Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceantinoside C, Yadanzioside N, und P, Isodeoxyelephantopin, Tomenphantopin A und B, Coronarin A,B,C und D, Ursolsäure, Hyptatsäure A, Iso-Iridogermanal, Maytenfoliol, Effusantin A, Excisanin A und B, Longikaurin B, Sculponeatin C, Kamebaunin, Leukamenin A und B, 13,18-Dehydro-6-alpha-senecioyloxychaparrin, Taxamairin A und B, Regenilol, Triptolid, Cymarin, Hydroxyanopterin, Protoanemonin, Cheliburinchlorid, Sinococulin A und B, Dihydronitidin, Nitidinchlorid, 12-beta-Hydroxypregnadien-3,20-dion, Helenalin, Indicin, Indicin-N-oxid, Lasiocarpin, Inotodiol, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Periplocosid A, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Dihydrousambaraensin, Hydroxyusambarin, Strychnopentamin, Strychnophyllin, Usambarin, Usambarensin, Liriodenin, Oxoushinsunin, Daphnoretin, Lariciresinol, Methoxylariciresinol, Syringaresinol, Sirolimus, Rapamycin, Somatostatin, Tacrolimus, Roxithromycin, Troleandomycin, Simvastatin, Rosuvastatin, Vinblastin, Vincristin, Vindesin, Teniposid, Vinorelbin, Tropfosfamid, Treosulfan, Tremozolomid, Thiotepa, Tretinoin, Spiramycin, Umbelliferon, Desacetylvismion A, Vismion A und B, Zeorin.

**10.** Verfahren gemäß Anspruch 9, wobei der mindestens eine antiproliferative, antiinflammatorische, antiphlogistische, zytostatische, zytotoxische, antiangiogene, antirestenotische oder antithrombotische Wirkstoff aus der Gruppe ausgewählt wird umfassend:

Paclitaxel, Derivate und Analog des Paclitaxel, 6-α-Hydroxy-Paclitaxel, 2'-Succinylpaclitaxel, 2'-Succinylpaclitaxeltriethanol-amin, 2'-Glutarylpaclitaxel, 2'-Glutarylpaclitaxeltriethanolamin, 2'-O-Ester von Paclitaxel mit N-(Dimethylaminoethyl)glutamid, 2'-O-Ester von Paclitaxel mit N-(Dimethylaminoethyl)glutamidhydrochlorid, Taxotere.

## Claims

**1.** Method for coating or filling the folds of a folded catheter balloon, comprising the following steps:

a) providing a composition comprising at least a contrast agent and at least an antiproliferative, antiinflammatory, antiphlogistic, cytostatic, cytotoxic, antiangiogenic, anti-restenotic or antithrombotic active agent and at least an alcohol as solvent;
b) inserting the composition comprising at least a contrast agent and at least an antiproliferative, antiinflammatory, antiphlogistic, cytostatic, cytotoxic, antiangiogenic, anti-restenotic or antithrombotic active agent and at least an alcohol as solvent selectively into the folds of the folded catheter balloon by means of a syringe device, the composition being delivered from the syringe device at the distal or proximal end of a fold of the folded catheter balloon and the fold being filled due to capillary forces;
c) drying the composition comprising at least a contrast agent and at least an antiproliferative, antiinflammatory, antiphlogistic, cytostatic, cytotoxic, antiangiogenic, anti-restenotic or antithrombotic active agent and at least an alcohol as solvent by removing the solvent via evaporation or under reduced pressure.

**2.** Method according to claim 1, wherein folded catheter balloons are coated or filled in the compressed or deflated state or at maximum 10 % inflated state.

**3.** Method according to claim 1 or 2, wherein two or more or all of the folds of the folded catheter balloon are filled or coated simultaneously.

**4.** Method according to any of claims 1 to 3, wherein selectively only the folds of the folded catheter balloon are coated

or filled and the surface of the folded catheter balloon outside the folds is not coated.

**5.** Method according to any of claims 1 to 4, wherein after the filling or coating of one or more folds the folded catheter balloon is rotated in the direction of the fold openings.

**6.** Method according to any of claims 1 to 5, wherein the composition contains at least an alcohol as solvent selected from the group comprising:

methanol, ethanol, isopropanol, n-butanol, iso-butanol, t-butanol, ethyleneglycol, propyleneglycol, 1,3-propanediol, butyleneglycol, 1,3-butanediol, 1,4-butanediol, glycerin, 1,2,3-butanetriol, 1,2,4-butanetriol and 1,2,3,4-butanetetraol.

**7.** Method according to any of claims 1 to 6, wherein the composition contains at least a contrast agent selected from the group comprising:

x-ray contrast agents, iodine-containing contrast agents, contrast agents having a 1,3,5-triiodobenzene nucleus, nephrotropic x-ray contrast agents having low osmolarity, amidotrizoic acid, iothalaminic acid, iotrolan, iopamidol, iodoxaminic acid, diatrizoic acid, iomeprol, iopromid, desmethoxyacetyl-iopromid (DAMI), 5-amino-2,4,6-triiodophthalic acid-(2,3-dihydroxypropyl)-amide (ATH).

**8.** Method according to any of claims 1 to 7, wherein the composition contains at least an oil and/or at least a fatty acid selected from the group comprising:

linseed oil, flax oil, hempseed oil, corn oil, walnut oil, rape oil, soy bean oil, sunflower oil, poppy-seed oil, safflower oil, wheat germ oil, thistle oil, grape-seed oil, evening primrose oil, borage oil, black cumin oil, algae oil, fish oil, cod-liver oil, taririnic acid, ximeninic acid, stearolinic acid, 6,9-octadeceninic acid, pyrulinic acid, crepenynic acid, heisterinic acid, ETYA, linoleic acid, Y-linoleic acid, dihomo-Y-linoleic acid, arachidonic acid, α-linolenic acid, stearidonic acid, EPA, DPA, DHA, meadic acid, stellaheptaenic acid, taxolic acid, pinolenic acid, sciadonic acid, myristoleic acid, palmitoleic acid, petroselinic acid, oleic acid, vaccenic acid, gadoleinic acid, gondoinic acid, erucinic acid, nervonic acid, elaidinic acid, t-vaccenic acid, laurinic acid, myristinic acid, palmitinic acid, margarinic acid, stearinic acid, arachinic acid, behenic acid, lignocerinic acid, 6,8-dithianoctanoic acid, Y-linolenic acid, α-liponic acid.

**9.** Method according to any of claims 1 to 5, wherein the at least one antiproliferative, antiinflammatory, antiphlogistic, cytostatic, cytotoxic, antiangiogenic, anti-restenotic or antithrombotic active agent is selected from the group comprising:

abciximab, acemetacin, acetylvismione B, aclarubicin, ademetionine, adriamycin, aescin, afromoson, akagerine, aldesleukin, amidorone, aminoglutethemide, amsacrine, anakinra, anastrozole, anemonin, anopterine, antimycotics, antithrombotics, apocymarin, argatroban, aristolactam-AlI, aristolochic acid, ascomycin, asparaginase, aspirin, atorvastatin, auranofin, azathioprine, azithromycin, baccatine, bafilomycin, basiliximab, bendamustine, benzocaine, berberine, betulin, betulinic acid, bilobol, biolimus, bisparthenolidine, bleomycin, bombrestatin, Boswellic acids and derivatives thereof, bruceanoles A, B and C, bryophyllin A, busulfan, antithrombin, bivalirudin, cadherins, camptothecin, capecitabine, o-carbamoylphenoxy-acetic acid, carboplatin, carmustine, celecoxib, cepharanthin, cerivastatin, CETP inhibitors, chlorambucil, chloroquine phosphate, cictoxin, ciprofloxacin, cisplatin, cladribine, clarithromycin, colchicine, concanamycin, coumadin, C-type Natriuretic Peptide (CNP), cudraisoflavone A, curcumin, cyclophosphamide, cyclosporine A, cytarabine, dacarbazine, daclizumab, dactinomycin, dapson, daunorubicin, diclofenac, 1,11-dimethoxycanthin-6-one, docetaxel, doxorubicin, dunaimycin, epirubicin, epothilones A and B, erythromycin, estramustine, etoposide, everolimus, filgrastim, fluoroblastin, fluvastatin, fludarabine, fludarabine-5'-dihydrogenphosphate, fluorouracil, folimycin, fosfestrol, gemcitabine, ghalakinoside, ginkgol, ginkgolic acid, glycoside 1A, 4-hydroxyoxycyclophosphamide, idarubicin, ifosfamide, josamycin, lapachol, lomustine, lovastatin, melphalan, midecamycin, mitoxantrone, nimustine, pitavastatin, pravastatin, procarbazin, mitomycin, methotrexate, mercaptopurine, thioguanine, oxaliplatin, irinotecan, topotecan, hydroxycarbamide, miltefosine, pentostatin, pegasparase, exemestane, letrozole, formestane, SMC proliferation inhibitor-2ω, mitoxantrone, mycophenolate mofetil, c-myc antisense, b-myc antisense, ß-lapachone, podophyllotoxin, podophyllic acid 2-ethylhydrazide, molgramostim (rhuGM-CSF), peginterferon α-2b, lanograstim (r-HuG-CSF), macrogol, selectin (cytokine antagonist), cytokinin inhibitors, COX-2 inhibitor, NFkB, angiopeptin, monoclonal antibodies which inhibit muscle cell proliferation, bFGF antagonists, probucol, prostaglandins, 1-hydroxy-11-methoxycanthin-6-one, scopolectin, NO donors, pentaerythrityl tetranitrate, sydnone imines, S-nitroso derivatives, tamoxifen, staurosporine, ß-estradiol, α-estradiol, estriol, estrone, ethinylestradiol, medroxyprogesterone, estradiol cypionates, estradiol benzoates, tranilast, kamebakaurin and other terpenoids which are applied in the therapy of cancer, verapamil, tyrosine kinase inhibitors (tyrphostins), paclitaxel, derivatives and analoga of paclitaxel, 6-α-hydroxy-paclitaxel, 2'-succinylpaclitaxel, 2'-succinylpaclitaxeltriethanolamine, 2'-glutarylpaclitaxel, 2'-glutarylpaclitaxeltriethanolamine, 2'-O-ester of paclitaxel with N-(dimethylaminoethyl)glutamide, 2'-O-ester of paclitaxel with N-(dimethylaminoethyl)glutamide hydrochloride, taxotere, carbon suboxide (MCS), macrocyclic oligomers of carbon suboxide, mofebutazone, lonazolac, lidocaine, ketoprofen, mefenamic acid, piroxicam, meloxicam, penicillamine, hydroxychloroquine, sodium aurothiomalate, oxaceprol, ß-sitosterin, myrtecaine, polidocanol, nonivamide, levomenthol, ellipticine, D-24851 (Calbiochem), colcemid, cytochalasin A-E, indanocine, nocadazole, S 100 protein, bacitracin, vitronectin receptor antagonists, azelastine, guanidyl cyclase stimulator tissue inhibitor of metal proteinase-1 and -2, free nucleic acids, nucleic acids incorporated into virus transmitters, DNA and RNA fragments, plasminogen activator inhibitor-1, plasminogen activator inhibitor-2, antisense oligonucleotides, VEGF inhibitors, IGF-1, active agents from the group of antibiotics, cefadroxil, cefazolin, cefaclor, cefoxitin, tobramycin, gentamycin, penicillins, dicloxacillin, oxacillin, sulfonamides, metronidazole, enoxoparin, desulphated and N-reacetylated heparin, tissue plasminogen activator, GpIIb/IIIa platelet membrane receptor, factor Xa inhibitor antibody, heparin, hirudin, r-hirudin, PPACK, protamine, prourokinase, streptokinase, warfarin, urokinase, vasodilators, dipyramidol, trapidil, nitroprussides, PDGF antagonists, triazolopyrimidine, seramin, ACD inhibitors, captopril, cilazapril, lisinopril, enalapril, losartan, thioprotease inhibitors, prostacyclin, vapiprost, interferon α, ß and Y, histamine antagonists, serotonin blockers, apoptosis inhibitors, apoptosis regulators, p65, NF-kB, Bcl-xL antisense oligonucleotides, halofuginone, nifedipine, tocopherol, tranilast, molsidomine, tea polyphenols, epicatechin gallate, epigallocatechin gallate, leflunomide, etanercept, sulfasalazine, etoposide, dicloxacillin, tetracycline, triamcinolone, mutamycin, procainimide, retinoic acid, quinidine, disopyramide, flecainide, propafenone, sotolol, natural and synthetically obtained steroids, inotodiol, maquiroside A, ghalakinoside, mansonine, strebloside, hydrocortisone, betamethasone, dexamethasone, non-steroidal substances (NSAIDS), fenoprofen, ibuprofen, indomethacin, naproxen, phenylbutazone, antiviral agents, acyclovir, ganciclovir, zidovudin, clotrimazole, flucytosine, griseofulvin, ketoconazole, miconazole, nystatin, terbinafine, antiprotozoal agents, chloroquine, mefloquine, quinine, natural terpenoids, hippocaesculin, barringtogenol-C21-angelat, 14-dehydroagrostistachin, agroskerin, agrostistachin, 17-hydroxyagrostistachin, ovatodiolids, 4,7-oxycycloanisomel acid, baccharinoids B1, B2, B3 and B7, tubeimoside, bruceantinoside C, yadanziosides N and P, isodeoxyelephantopin, tomenphantopin A and B, coronarin A, B, C and D, ursolic acid, hyptatic acid A, iso-iridogermanal, maytenfoliol, effusantin A, excisanin A and B, longikaurin B, sculponeatin C, kamebaunin, leukamenin A and B, 13,18-dehydro-6-alpha-senecioyloxychaparrin, taxamairin A and B, regenilol, triptolide, cymarin, hydroxyanopterin, protoanemonin, cheliburin chloride, sinococuline A and B, dihydronitidine, nitidine chloride, 12-beta-hydroxypregnadien-3,20-dion, helenalin, indicine, indicine-N-oxide, lasiocarpine, inotodiol, podophyllotoxin, justicidin A and B, larreatin, malloterin, mallotochromanol, isobutyrylmallotochromanol, maquiroside A, marchantin A, maytansin, lycoridicin, margetine, pancratistatin, liriodenine, bisparthenolidine, oxoushinsunine, periplocoside A, ursolic acid, deoxypsorospermin, psycorubin, ricin A, sanguinarine, manwu wheat acid, methylsorbifolin, sphateliachromen, stizophyllin, mansonine, strebloside, dihydrousambaraensine, hydroxyusambarine, strychnopentamine, strychnophylline, usambarine, usambarensine, liriodenine, oxoushinsunine, daphnoretin, lariciresinol, methoxylariciresinol, syringaresinol, sirolimus, rapamycin, somatostatin, tacrolimus, roxithromycin, troleandomycin, simvastatin, rosuvastatin, vinblastine, vincristine, vindesine, teniposide, vinorelbine, tropfosfamide, treosulfan, temozolomide, thiotepa, tretinoin, spiramycin, umbelliferone, desacetylvismione A, vismione A and B, zeorin.

**10.** Method according to claim 9, wherein the at least one antiproliferative, antiinflammatory, antiphlogistic, cytostatic, cytotoxic, antiangiogenic, anti-restenotic or antithrombotic active agent is selected from the group comprising:

paclitaxel, derivatives and analoga of paclitaxel, 6-α-hydroxy-paclitaxel, 2'-succinylpaclitaxel, 2'-succinylpaclitaxeltriethanolamine, 2'-glutarylpaclitaxel, 2'-glutarylpaclitaxeltriethanolamine, 2'-O-ester of paclitaxel with N-(dimethylaminoethyl)glutamide, 2'-O-ester of paclitaxel with N-(dimethylaminoethyl)glutamide hydrochloride, taxotere.

## Revendications

**1.** Procédé pour revêtir ou remplir les plis d'un ballonnet repliable de cathéter, comprenant les étapes suivantes :

a) fourniture d'une composition comprenant au moins un produit de contraste et au moins un principe actif antiprolifératif, anti-inflammatoire, antiphlogistique, cytostatique, cytotoxique, antiangiogénique, antiresténotique ou antithrombotique, et au moins un alcool en tant que solvant ;

b) introduction d'une manière ciblée, à l'aide d'un dispositif d'injection, dans les plis du ballonnet repliable de cathéter, de la composition comprenant au moins un produit de contraste et au moins un principe actif antiprolifératif, anti-inflammatoire, antiphlogistique, cytostatique, cytotoxique, antiangiogénique, antiresténotique ou antithrombotique et au moins un alcool en tant que solvant, la composition étant évacuée du dispositif d'injection à l'extrémité distale ou proximale d'une fente du ballonnet de cathéter, et les plis étant remplis sous l'effet de forces capillaires ;

c) séchage par élimination du solvant par évaporation ou sous pression réduite de la composition comprenant au moins un produit de contraste et au moins un principe actif antiprolifératif, anti-inflammatoire, antiphlogistique, cytostatique, cytotoxique, antiangiogénique, antiresténotique ou antithrombotique, et au moins un alcool en tant que solvant.

**2.** Procédé selon la revendication 1, dans lequel on revêt ou remplit des ballonnets repliables de cathéter à l'état comprimé ou dégonflé, ou en un état gonflé au maximum à 10 %.

**3.** Procédé selon la revendication 1 ou 2, dans lequel on remplit ou revêt simultanément deux ou plus, ou la totalité des plis du ballonnet repliable de cathéter.

**4.** Procédé selon l'une des revendications 1 à 3, dans lequel on revêt ou remplit d'une manière ciblée uniquement les plis du ballonnet repliable de cathéter, la surface du ballonnet repliable de cathéter à l'extérieur des plis n'étant pas revêtue.

**5.** Procédé selon l'une des revendications 1 à 4, dans lequel, après le remplissage ou le revêtement d'un ou plusieurs plis, on fait tourner le ballonnet repliable de cathéter dans la direction de l'ouverture des plis.

**6.** Procédé selon l'une des revendications 1 à 5, dans lequel la composition contient au moins un alcool en tant que solvant, choisi dans le groupe comprenant le méthanol, l'éthanol, l'isopropanol, le n-butanol, l'isobutanol, le tert-butanol, l'éthylèneglycol, le propylèneglycol, le 1,3-propanediol, le butylèneglycol, le 1,3-butanediol, le 1,4-butanediol, le glycérol, le 1,2,3-butanetriol, le 1,2,4-butanetriol et le 1,2,3,4-butanetétraol.

**7.** Procédé selon l'une des revendications 1 à 6, dans lequel la composition contient au moins un produit de contraste choisi dans le groupe comprenant les produits de contraste aux rayons X, les produits de contraste iodés, les produits de contraste ayant un noyau 1,3,5-triiodobenzène, les produits de contraste aux rayons X néphrotropiques de basse osmolalité, l'acide amidotrizoïque, l'acide iothalamique, l'iotrolan, l'iopamidol, l'acide iodoxamique, l'acide diatrizoïque, l'ioméprol, l'iopromide, le desméthoxyacétyl-iopromide (DAMI), le (2,3-dihydroxypropyl)-amide de l'acide 5-amino-2,4,6-triiodophtalique (ATH).

**8.** Procédé selon l'une des revendications 1 à 7, dans lequel la composition contient au moins une huile et/ou au moins un acide gras choisi dans le groupe comprenant : l'huile de lin, l'huile de graine de lin, l'huile de chènevis, l'huile de germe de maïs, l'huile de noix, l'huile de colza, l'huile de soja, l'huile de tournesol, l'huile d'oeillette, l'huile de carthame (l'huile de carthame des teinturiers), l'huile de germe de blé, l'huile de chardon, l'huile de pépins de raisin, l'huile d'onagre, l'huile de bourrache, l'huile de nigelle, l'huile d'algues, l'huile de poisson, l'huile de foie de morue, l'acide tariríque, l'acide ximénique, l'acide stéarolique, l'acide 6,9-octadécénoïque, l'acide pyrulique, l'acide crépénynique, l'acide héistérique, l'ETYA, l'acide linoléique, l'acide $\gamma$-linoléique, l'acide dihomo-$\gamma$-linolénique, l'acide arachidonique, l'acide $\alpha$-linolénique, l'acide stéaridonique, l'EPA, le DPA, le DHA, l'acide de Mead, l'acide stellaheptaénoïque, l'acide taxolique, l'acide pinolénique, l'acide sciadonique, l'acide myristoléique, l'acide palmitoléique, l'acide pétrosélinique, l'acide oléique, l'acide vaccénique, l'acide gadoléique, l'acide gondoïnique, l'acide érucique, l'acide nervonique, l'acide élaïdique, l'acide t-vaccénique, l'acide laurique, l'acide myristique, l'acide palmitique, l'acide margarique, l'acide stéarique, l'acide arachidique, l'acide béhonique, l'acide lignocérique, l'acide 6,8-dithianooctanoïque, l'acide $\gamma$-linolénique, l'acide $\alpha$-lipoïque.

**9.** Procédé selon l'une des revendications 1 à 5, dans lequel le ou les principes actifs antiprolifératifs, anti-inflammatoires, antiphlogistiques, cytostatiques, cytotoxiques, antiangiogéniques, antiresténotiques ou antithrombotiques sont choisis dans le groupe comprenant : l'abciximab, l'acémétacine, l'acétylvismion B, l'aclarubicine, l'adémétionine, l'adriamycine, l'aescine, l'afromosone, l'akagérine, l'aldesleukine, l'amidorone, l'aminoglutéthémide, l'amsacrine, l'anakinra, l'anastrozol, l'anémonine, l'anoptérine, les antimycotiques, les antithrombotiques, l'apocymarine,

l'argatroban, l'aristolactame-AII, l'acide aristolochique, l'ascomycine, l'asparaginase, l'aspirine, l'atorvastatine, l'auranofine, l'azathioprine, l'azithromycine, la baccatine, la bafilomycine, le basiliximab, la bendamustine, la benzocaïne, la berbérine, la bétuline, l'acide bétulinique, le bilobol, le Biolimus, la bisparthénolidine, la bléomycine, la bombrestatine, l'acide boswellique et ses dérivés, les brucéanols A, B et C, la bryophylline A, le busulfan, l'antithrombine, la bivalirudine, la cadhérine, la camptothécine, la capécitabine, l'acide o-carbamoylphénoxyacétique, le carboplatine, la carmustine, le célécoxib, la cépharantine, la cérivastatine, les inhibiteurs du CETP, le chlorambucil, le phosphate de chloroquine, la cictoxine, la ciprofloxacine, le cisplatine, la cladribine, la clarithromycine, la colchicine, la concanamycine, la coumadine, le peptide natriurétique de type C (CNP), la cudraisoflavone A, la curcumine, le cyclophosphamide, la cyclosporine A, le cytarabine, la dacarbazine, le daclizumab, la dactinomycine, la dapsone, la daunorubicine, le diclofénac, la 1,11-diméthoxycanthin-6-one, le docétaxel, la doxorubicine, la dunaïmycine, l'épirubicine, les épothilones A et B, l'érythromycine, l'estramustine, l'étoboside, l'évérolimus, le filgrastim, la fluroblastine, la fluvastatine, la fludarabine, le 5'-dihydrogénophosphate de fludarabine, le fluorouracile, la folimycine, le fosfestrol, la gemcitabine, le ghalakinoside, le ginkgol, l'acide ginkgolique, le glycoside 1a, le 4-hydroxyoxycyclophosphamide, l'idarubicine, l'ifosfamide, la josamycine, le lapachol, la lomustine, la lovastatine, le melphalan, la midécamycine, la mitoxantrone, la nimustine, la pitavastatine, la pravastatine, la procarbazine, la mitomycine, le méthotrexate, la mercaptopurine, la thioguanine, l'oxaliplatine, l'irinotécan, le topotécan, l'hydroxycarbamide, la miltéfosine, la pentostatine, la pégasparase, l'exemestan, le létrozol, le formestan, l'inhibiteur 2$\omega$ de la prolifération des CML, les mitoxanthrones, le mycophénolatmofétil, le c-myc-antisens, le b-myc-antisens, la $\beta$-lapachone, la podophyllotoxine, le 2-éthylhydrazide de l'acide podophyllique, le molgramostim (rhuGM-CSF), le péginterféron $\alpha$-2b, le lanograstim (r-HuG-CSF), le macrogol, la sélectine (antagoniste des cytokines), les inhibiteurs des cytokines, l'inhibiteur de COX-2, le NFkB, l'angiopeptine, les anticorps monoclonaux inhibiteurs de la prolifération des cellules musculaires, les antagonistes du bFGF, le probucol, les prostaglandines, la 1-hydroxy-11-méthoxycanthin-6-one, la scopolectine, les donneurs de NO, le tétranitrate de pentaérythrityle, les syndnoeimines, les dérivés S-nitrosés, le tamoxifène, la staurosporine, le $\beta$-estradiol, l'$\alpha$-estradiol, l'estriol, l'estrone, l'éthynylestradiol, la médroxyprogestérone, le cypionate d'estradiol, le benzoate d'estradiol, le tranilast, la kamébakaurine et d'autres terpénoïdes qui sont utilisés dans le traitement du cancer, le vérapamil, les inhibiteurs de tyrosine-kinase (tyrphostines), le paclitaxel, les dérivés et analogues du paclitaxel, le 6-$\alpha$-hydroxy-paclitaxel, le 2'-succinylpaclitaxel, la 2'-succinylpaclitaxeltriéthanolamine, le 2'-glutarylpaclitaxel, la 2'-glutarylpaclitaxeltriéthanolamine, le 2'-O-ester du paclitaxel et du N-(diméthylaminoéthyl)glutamide, le 2'-O-ester du paclitaxel et du chlorhydrate de N-(diméthylaminoéthyl)glutamide, les taxotères, le sous-oxyde de carbone (MCS), les oligomères macrocycliques du sous-oxyde de carbone, la mofébutazone, le lonazolac, la lidocaïne, le kétoprofène, l'acide méfénamique, le piroxicam, le méloxicam, la pénicillamine, l'hydroxychloroquine, l'aurothiomalate de sodium, l'oxacéprol, la $\beta$-sitostérine, la myrtécaïne, le polidocanol, le nonivamide, le lévomenthol, l'ellipticine, le D-24851 (Calbiochem), le colcémide, la chytochalasine A-E, les indanocines, les nocadazoles, la protéine S100, la bacitracine, les antagonistes du récepteur de la vitronectine, l'azélastine, le stimulateur de guanidylcyclase, l'inhibiteur tissulaire de la métalloprotéinase-1 et -2, les acides nucléiques libres, les acides nucléiques incorporés dans les vecteurs viraux, les fragments d'ADN et d'ARN, l'inhibiteur-1 de l'activateur du plasminogène, l'inhibiteur-2 de l'activateur du plasminogène, les oligonucléotides antisens, les inhibiteurs du VEGF, l'IGF-1, les principes actifs du groupe des antibiotiques, le céfadroxil, la céfazoline, le céfaclor, la céfotixine, la tobramycine, la gentamycine, les pénicillines, la dicloxacilline, l'oxacilline, les sulfonamides, le métronidazol, l'énoxoparine, l'héparine désulfatée et N-réacétylée, l'activateur du plasminogène tissulaire, le récepteur membranaire plaquettaire GpIIb/IIIa, les anticorps des inhibiteurs du facteur Xa, l'héparine, l'hirudine, la r-hirudine, le PPACK, la protamine, la prourokinase, la streptokinase, la warfarine, l'urokinase, les vasodilatateurs, le dipyramidol, le trapidil, le nitroprusside, les antagonistes du PDGF, la triazolopyrimidine, la séramine, les inhibiteurs de l'ECA, le captopril, le cilazapril, le lisinopril, l'énalapril, le losartan, les inhibiteurs des thioprotéases, la prostacycline, le vapiprost, l'interféron a, $\beta$ et $\gamma$, les antagonistes des histamines, les sérotonino-bloquants, les inhibiteurs de l'apoptose, les régulateurs de l'apoptose, le p65, le NF-kB, les oligonucléotides Bcl-xL-antisens, l'halofuginone, la nifédipine, le tocophérol, le tranilast, la molsidomine, les polyphénols du thé, le gallate d'épicatéchine, le gallate d'épigallocatéchine, le léflunomide, l'étanercept, la sulfasalazine, l'étoposide, la dicloxacylline, la tétracycline, la triamcinolone, la mutamycine, le procaïnimide, l'acide rétinoïque, la quinidine, le disopyrimide, le flécaïnide, la propafénone, le sotolol, les stéroïdes naturels et fabriqués par synthèse, l'inotodiol, le maquiroside A, le ghalakinoside, la mansonine, le strébloside, l'hydrocortisone, la bétaméthasone, la dexaméthasone, les substances non stéroïdiennes (AINS), le fénoporfène, l'ibuprofène, l'indométhacine, le naproxène, la phénylbutazone, les agents antiviraux, l'acyclovir, le ganciclovir, la zidovudine, le clotrimazol, la flucytosine, la griséofulvine, le kétoconazol, le miconazol, la nystatine, la terbinafine, les agents antiprozoaires, la chloroquine, la méfloquine, la quinine, les terpénoïdes naturels, l'hippocaesculine, l'angélate de barringtogénol-C21, la 14-déhydroagrostistachine, l'agroskérine, l'agrostistachine, la 17-hydroxyagrostistachine, les ovatodiolides, l'acide 4,7-oxycycloanisomélique, les baccharinoïdes B1, B2, B3 et B7, le tubéimoside, les brucéantinosides C, les yadanziosides N et P, l'isodésoxyéléphantopine, la tomenphantopine A et B, la coronarine A,B,C et D, l'acide ursolique, l'acide hyptatique A, l'iso-iridogermanal, le

maytenfoliol, l'effusantine A, l'excisanine A et B, la longikaurine B, la sculponéatine C, la kamébaunine, la leukaménine A et B, la 13,18-déhydro-6-alpha-sénocioyloxychaparrine, la taxamairine A et B, le régénilol, le triptolide, la cymarine, l'hydroxyanoptérine, la protoanémonine, le chlorure de chéliburine, la sinococuline A et B, la dihydronitidine, le chlorure de nitidine, la 12-bêta-hydroxypregnadièn-3,20-dione, l'hélénaline, l'indicine, le N-oxyde d'indicine, la lasiocarpine, l'inotodiol, la podophyllotoxine, la justicidine A et B, la larréatine, la mallotérine, le mallotochromanol, l'isobutyrylmallotochromanol, le maquiroside A, la marchantine A, la maytansine, la lycoridicine, la margétine, la pancratistatine, la liriodénine, la bisparthénolidine, l'oxoushinsunine, le périplocoside A, l'acide ursolique, la désoxypsorospermine, la psycorubine, la ricine A, la sanguinarine, l'acide manwuwéizique, la méthylsorbifoline, le sphathéliachromène, la stizophylline, la mansonine, le strébloside, la dihydrousambaraensine, l'hydroxyusambarine, la strychnopentamine, la strychnophylline, l'usambarine, l'usambarensine, la liriodénine, l'oxoushinsunine, la daphnorétine, le laricirésinol, le méthoxylaricirésinol, le syringarésinol, le sirolimus, la rapamycine, la somatostatine, le tacrolimus, la roxithromycine, la troléandomycine, la simvastatine, la rosuvastatine, la vinblastine, la vincristine, la vindésine, le téniposide, la vinorelbine, le tropfosfamide, le tréosulfan, le trémozolomide, le thiotépa, la trétinoïne, la spiramycine, l'umbelliférone, la désacétylvismione A, la vismione A et B, la zéorine.

**10.** Procédé selon la revendication 9, dans lequel le ou les principes actifs antiprolifératifs, anti-inflammatoires, antiphlogistiques, cytostatiques, cytotoxiques, antiangiogéniques, antiresténotiques ou antithrombotiques, sont choisis dans le groupe comprenant le paclitaxel, les dérivés et l'analogue du paclitaxel, le 6-a-hydroxy-paclitaxel, le 2'-succinylpaclitaxel, la 2'-succinylpaclitaxeltriéthanolamine, le 2'-glutarylpaclitaxel, la 2'-glutarylpaclitaxeltriéthanolamine, le 2'-O-ester du paclitaxel et du N-(diméthylaminoéthyl)glutamide, le 2'-O-ester du paclitaxel et du chlorhydrate de N-(diméthylaminoéthyle)glutamide, les taxotères.

Figur 1

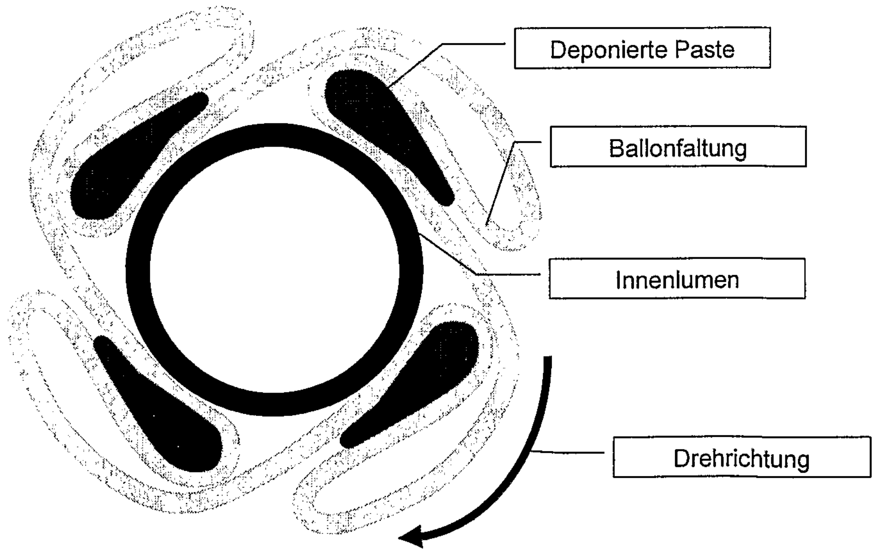
Deponierte Paste
Ballonfaltung
Innenlumen
Drehrichtung

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20040071861 A
- WO 03035131 A
- WO 0245744 A
- EP 0519063 B1
- WO 2004028582 A1
- WO 9423787 A1
- WO 03059430 A1
- WO 03022265 A1